(19)

Europäisches
Patentamt
European
Patent Office
Office européen
des brevets

(11) **EP 4 607 194 A1**

(12) **EUROPEAN PATENT APPLICATION**
published in accordance with Art. 153(4) EPC

(43) Date of publication:
**27.08.2025 Bulletin 2025/35**

(21) Application number: **23879883.9**

(22) Date of filing: **19.10.2023**

(51) International Patent Classification (IPC):
**G01N 33/53** *(2006.01)*          **A61F 13/42** *(2006.01)*
**A61F 13/511** *(2006.01)*        **G01N 33/543** *(2006.01)*

(52) Cooperative Patent Classification (CPC):
**A61F 13/15; A61F 13/42; A61F 13/511;
A61F 13/537; G01N 33/50; G01N 33/53;
G01N 33/543**

(86) International application number:
**PCT/JP2023/037940**

(87) International publication number:
**WO 2024/085240 (25.04.2024 Gazette 2024/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.10.2022  JP 2022169530
17.05.2023  PCT/JP2023/018507**

(71) Applicant: **Unicharm Corporation
Shikokuchuo-shi, Ehime 799-0111 (JP)**

(72) Inventors:
• **SUZUKI, Yuya
Kanonji-shi, Kagawa 769-1602 (JP)**

• **NOMOTO, Takashi
Kanonji-shi, Kagawa 769-1602 (JP)**
• **HASHINO, Akira
Kanonji-shi, Kagawa 769-1602 (JP)**
• **YOSHIMASA, Wataru
Kanonji-shi, Kagawa 769-1602 (JP)**
• **GODA, Hiroki
Kanonji-shi, Kagawa 769-1602 (JP)**
• **UEDA, Takahiro
Kanonji-shi, Kagawa 769-1602 (JP)**
• **NISHIMURA, Kiyoko
Kanonji-shi, Kagawa 769-1602 (JP)**
• **MIZUTANI, Sachi
Kanonji-shi, Kagawa 769-1602 (JP)**

(74) Representative: **Dolleymores
9 Rickmansworth Road
Watford, Hertfordshire WD18 0JU (GB)**

(54) **ABSORBENT ARTICLE HAVING VAGINAL DISCHARGE TEST STRIP, AND VAGINAL DISCHARGE TEST STRIP FOR USE IN ABSORBENT ARTICLE**

(57)    The present invention provides an absorbent article with which the day of ovulation can be predicted in a more convenient manner than conventionally, and relates to an absorbent article, comprising a test strip for detecting luteinizing hormone from vaginal discharge using immunochromatography, wherein the test strip detects 0.05 mIU/mL to 2.5 mIU/mL of luteinizing hormone as positive.

EP 4 607 194 A1

# Fig. 3-1

**Description**

FIELD

The present invention relates to an absorbent article comprising a vaginal discharge test strip. Furthermore, the present invention relates to a vaginal discharge test strip for use in an absorbent article. Note that the present disclosure includes an invention B. The invention B will be described later.

BACKGROUND

Identifying the day of ovulation in advance is important for women hoping to become pregnant. Testing for luteinizing hormone (LH) in urine is widely used as a method for predicting the day of ovulation.
Lateral flow assays for detecting the presence of an analyte in a liquid sample are also widely known (Patent Literature 1).
An absorbent article having a detection area based on immunochromatography is well known (Patent Literature 2). According to this absorbent article, a health condition of an individual can be understood by detecting components in human excretion components via an antigen-antibody reaction.

[CITATION LIST]

[PATENT LITERATURE]

[PTL 1] Japanese Unexamined PCT Publication (Kohyo) No. 2007-526443
[PTL 2] WO 2021/132724

SUMMARY

[TECHNICAL PROBLEM]

Conventional methods for predicting the day of ovulation which use urine as a detection target have problems regarding convenience and the time and effort required to perform the detection operation and obtain results.
An object of the present invention is to provide an absorbent article with which the day of ovulation can be predicted in a more convenient manner than conventionally.

[SOLUTION TO PROBLEM]

The object of the present invention can be achieved by the present invention, including the following aspects.

<Aspect 1>

An absorbent article, comprising a test strip for detecting luteinizing hormone from vaginal discharge using immunochromatography, wherein
the test strip detects 0.05 mIU/mL to 2.5 mIU/mL of luteinizing hormone as positive.

<Aspect 2>

The absorbent article according to Aspect 1, wherein the test strip has a width of 5 mm or less.

<Aspect 3>

The absorbent article according to Aspect 1, wherein the test strip has a width of 3 mm or less.

<Aspect 4>

The absorbent article according to any one of Aspects 1 to 3, wherein the test strip comprises:

a first member which contacts vaginal discharge,

a second member which contains a labeled antibody that recognizes luteinizing hormone in vaginal discharge, and

a third member which has a display part which contains a capture antibody,

the capture antibody is capable of capturing a complex formed by binding of the labeled antibody and luteinizing hormone, and

the test strip is configured such that the luteinizing hormone in vaginal discharge moves from the first member, through the second member, to the display part of the third member, and that

the display part of the third member changes color when the capture antibody captures the complex.

<Aspect 5>

The absorbent article according to Aspect 4, wherein the first member, the second member, and the third member are stacked in this order at least partially overlapping each other, and

a sum of thicknesses of the first member, the second member, and the third member in the stacking direction is 1.5 mm or less.

<Aspect 6>

The absorbent article according to Aspect 4 or 5, wherein the first member is composed of fibers having an average fiber diameter of 0.5 to 5 $\mu$m,

the second member is composed of fibers having an average fiber diameter of 10 to 40 $\mu$m, and

an average fiber diameter of the fibers of the first member is less than an average fiber diameter of the fibers of the second member.

<Aspect 7>

The absorbent article according to any one of Aspects 4 to 6, wherein an average fiber diameter R1 of the fibers constituting the first member and an average fiber diameter R2 of the fibers constituting the second member satisfy the relationship $5 \leq (R2/R1) \leq 20$.

<Aspect 8>

The absorbent article according to any one of Aspects 4 to 7, which satisfies at least one of the following:

(a) the second member has a basis weight of 100 gsm or more,

(b) a sum of the thicknesses of the first member, the second member, and the third member is 1.5 mm or less, and the thickness of the second member accounts for 30% or more of this sum of the thicknesses, and

(b) the second member has a density of 0.05 to 0.80 g/m$^3$.

<Aspect 9>

The absorbent article according to any one of Aspects 4 to 8, wherein the labeled antibody comprises a colorant having a particle diameter of 10 nm to 500 nm.

<Aspect 10>

The absorbent article according to any one of Aspects 4 to 8, wherein the labeled antibody comprises a colorant having a particle diameter of 250 nm to 500 nm.

<Aspect 11>

The absorbent article according to any one of Aspects 1 to 10, wherein the absorbent article comprises a top sheet, and when the absorbent article is used, the vaginal discharge reaches the test strip through the top sheet.

<Aspect 12>

The absorbent article according to Aspect 11, wherein the detection is performed by directly contacting at least a portion of the top sheet with a vulva.

<Aspect 13>

The absorbent article according to Aspect 11 or 12, wherein a basis weight of the top sheet is 20 gsm or less.

<Aspect 14>

The absorbent article according to any one of Aspects 11 to 13, wherein a thickness of the top sheet is 1 mm or less.

<Aspect 15>

The absorbent article according to any one of Aspects 11 to 14, wherein a single fiber diameter of fibers constituting the top sheet is 8.0 dtex or less.

<Aspect 16>

The absorbent article according to Aspect 11, wherein a basis weight of the top sheet is 5 gsm or more, a thickness of the top sheet is 0.1 mm or more, and a single fiber diameter of fibers constituting the top sheet is 1.0 dtex or more.

<Aspect 17>

The absorbent article according to any one of Aspects 11 to 16, wherein the top sheet at least partially has an embossed pattern, and a width of the test strip is greater than a size and/or spacing of each pattern of the embossed pattern.

<Aspect 18>

The absorbent article according to any one of Aspects 11 to 17, wherein the top sheet partially has an embossed pattern, and the test strip is capable of contacting the vaginal discharge through an area of the top sheet which does not have the embossed pattern.

<Aspect 19>

The absorbent article according to any one of Aspects 11 to 18, wherein the top sheet does not contain TiO2.

<Aspect 20>

The absorbent article according to any one of Aspects 11 to 19, wherein the test strip comprises a first member which contacts vaginal discharge, the first member is arranged in a position which faces a vaginal part of a wearer when worn, and
an average fiber diameter of fibers constituting the sample pad is equal to or less than an average fiber diameter of fibers constituting the top sheet.

<Aspect 21>

A test strip for an absorbent article for detecting luteinizing hormone in vaginal discharge using immunochromatography, wherein
0.05 mIU/mL to 2.5 mIU/mL of luteinizing hormone is detected as positive.

[ADVANTAGEOUS EFFECTS OF THE INVENTION]

According to the present invention, an absorbent article with which the day of ovulation can be predicted in a more convenient manner than conventionally can be provided.

BRIEF DESCRIPTION OF DRAWINGS

FIG. 1 is a photograph showing the results of a sensitivity test of test strips according to the present invention.
FIG. 2 is a photograph showing the results of a color intensity test of test strips according to the present invention.
FIG. 3-1 is a schematic plan view of an absorbent article according to an embodiment as viewed from the skin side.
FIG. 3-2 is a conceptual diagram showing a specific configuration of a test strip according to the present invention.
FIG. 3-3 is a schematic cross-sectional view of an absorbent article according to an aspect of the present invention.

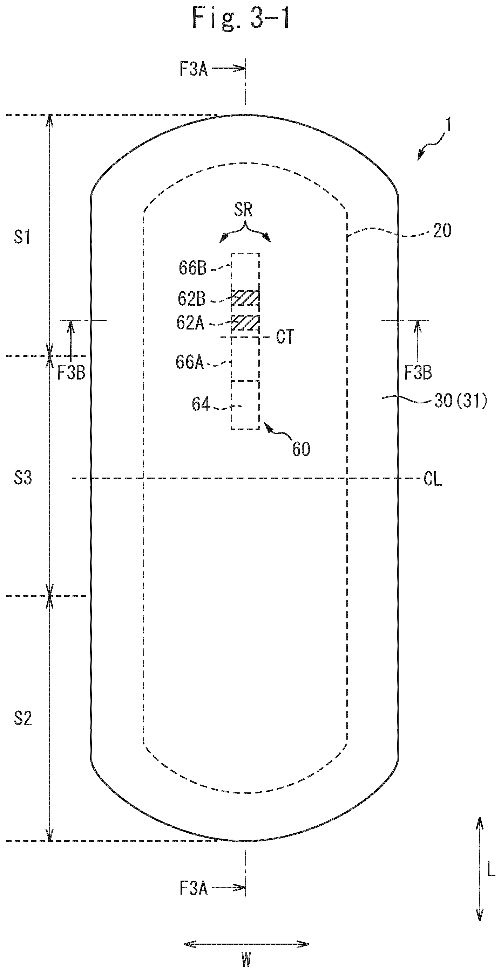

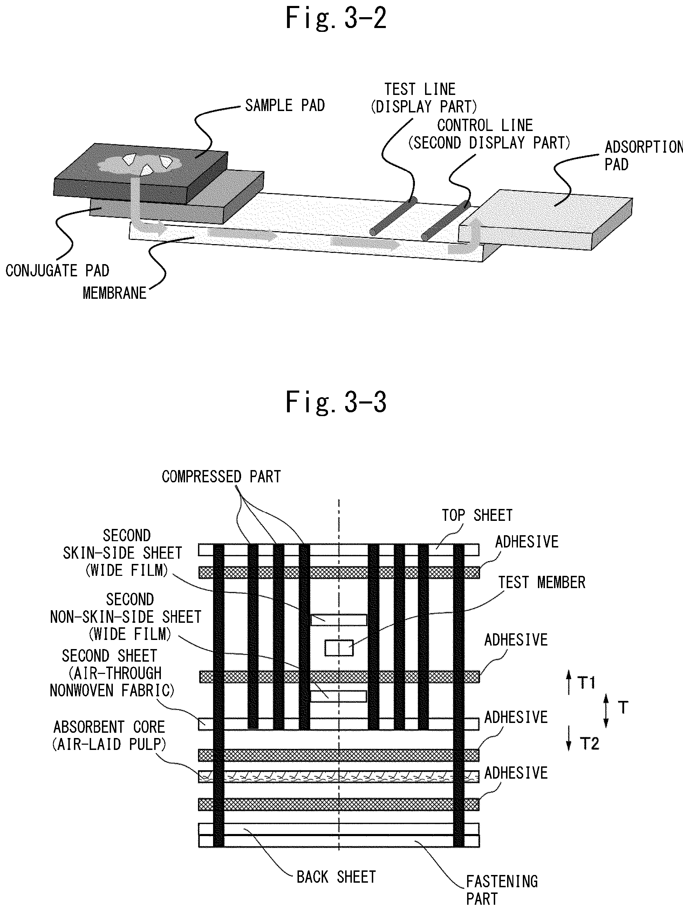

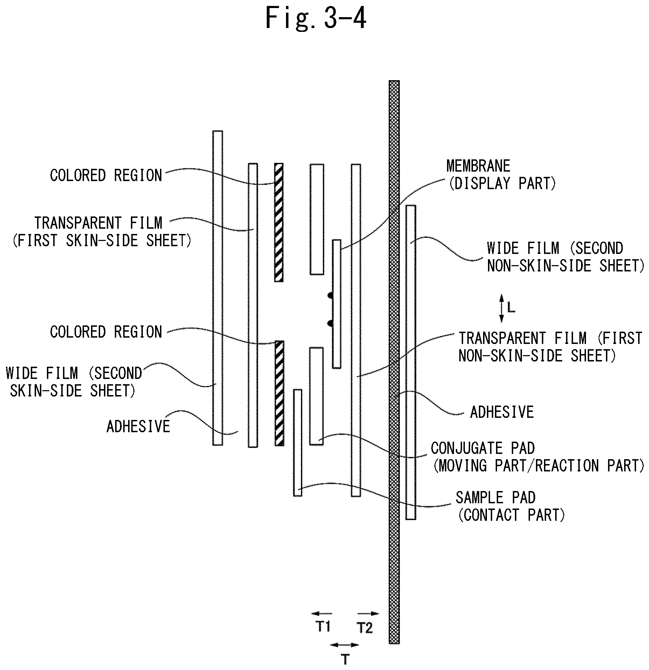

FIG. 3-4 is a schematic cross-sectional view of an absorbent article according to an aspect of the present invention.

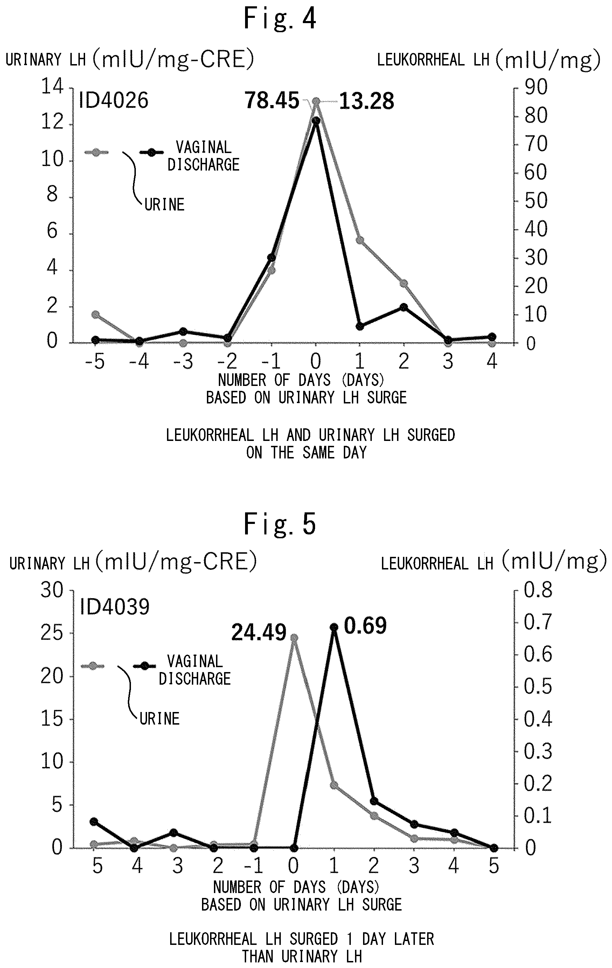

FIG. 4 is a graph plotting urinary LH concentration and leukorrheal LH concentration relative to the number of days based on the urinary LH surge.
FIG. 5 is a graph plotting urinary LH concentration and leukorrheal LH concentration relative to the number of days based on the urinary LH surge.

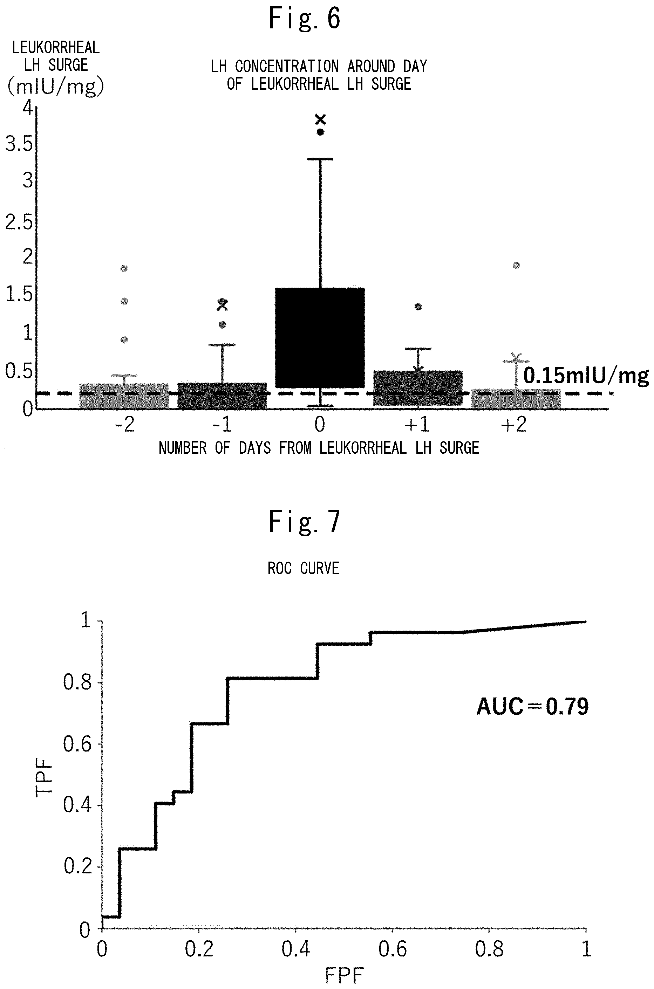

FIG. 6 is a graph showing LH concentration around the day of leukorrheal LH surge.
FIG. 7 is a graph showing the results of ROC analysis.

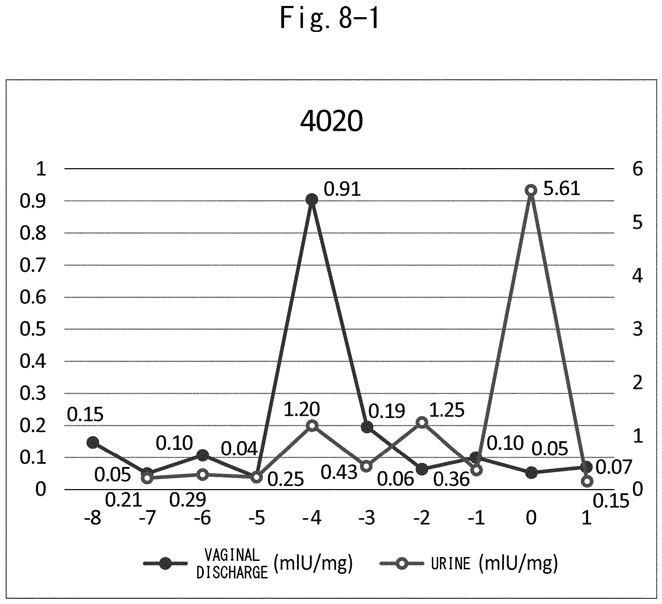

FIG. 8-1 is a graph showing LH surge in vaginal discharge.

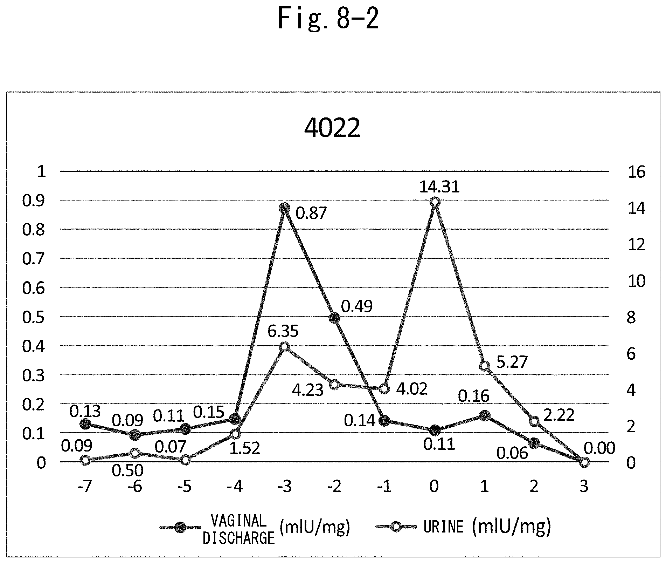

FIG. 8-2 is a graph showing LH surge in vaginal discharge.

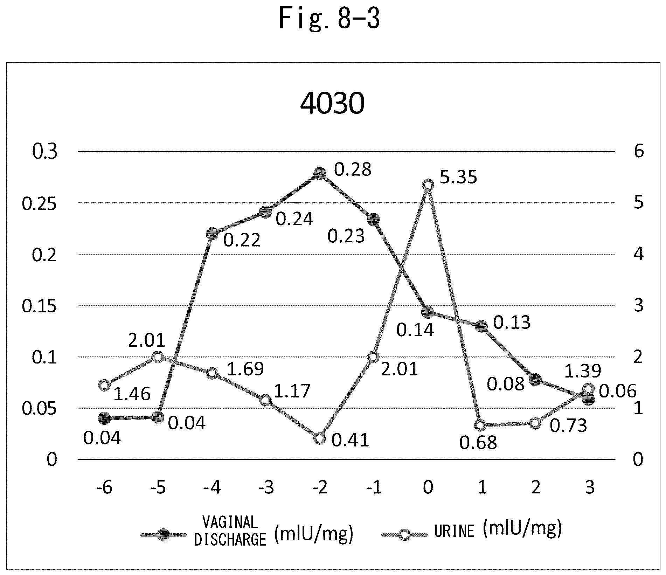

FIG. 8-3 is a graph showing LH surge in vaginal discharge.

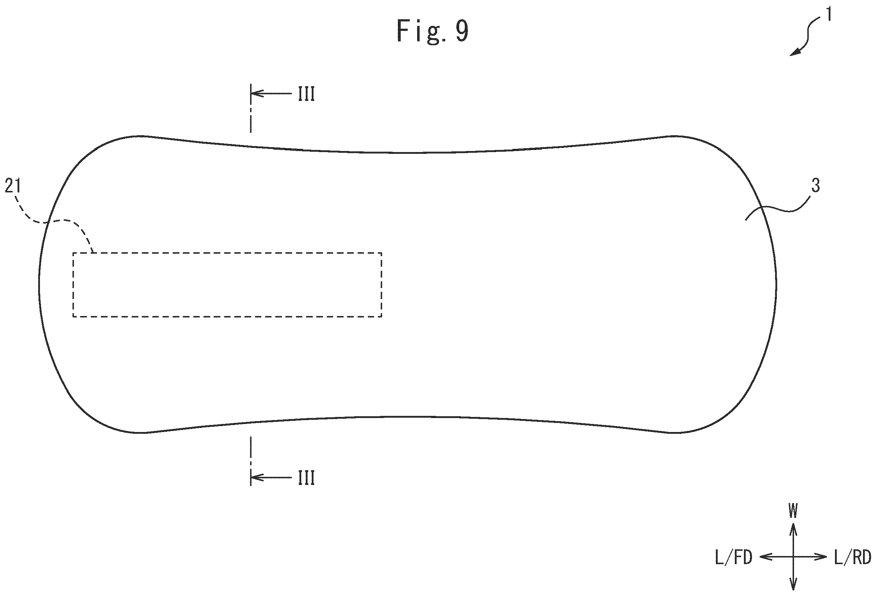

FIG. 9 is a plan view of a top sheet 3 side of a panty liner 1 according to a first embodiment of the present invention B.

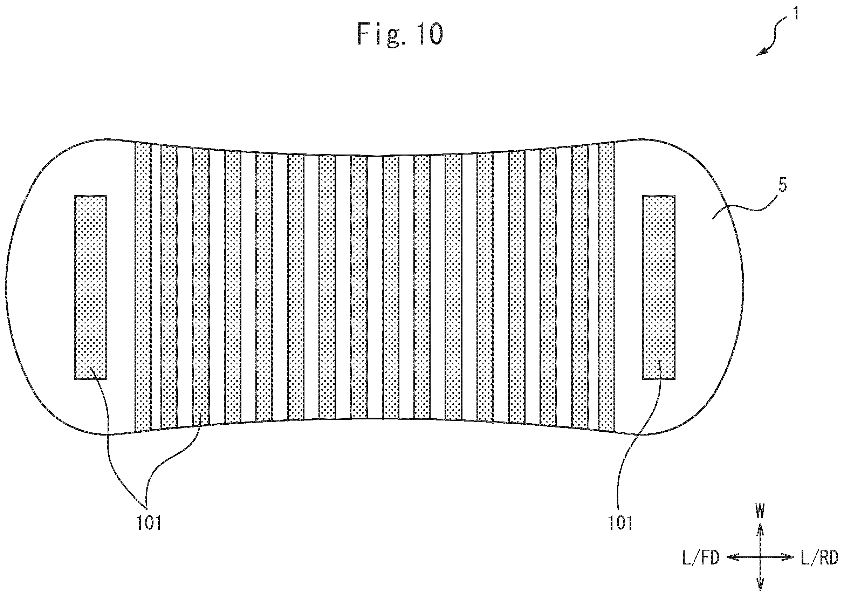

FIG. 10 is a plan view of a back sheet 5 side of the panty liner 1 according to the first embodiment of the present invention B.

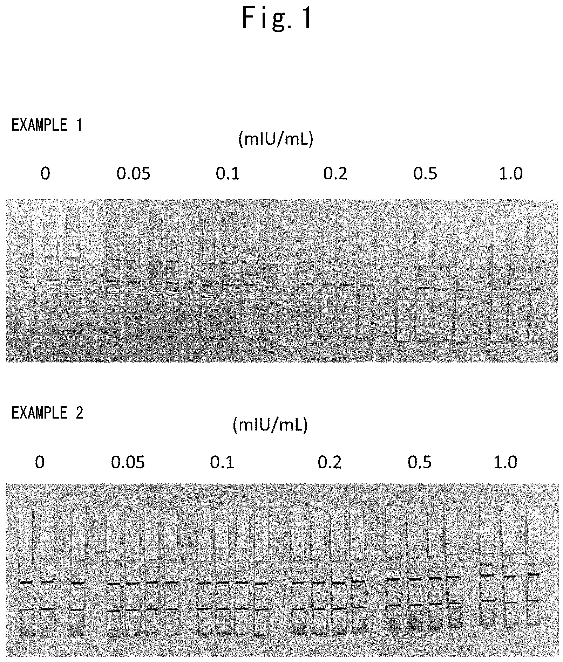

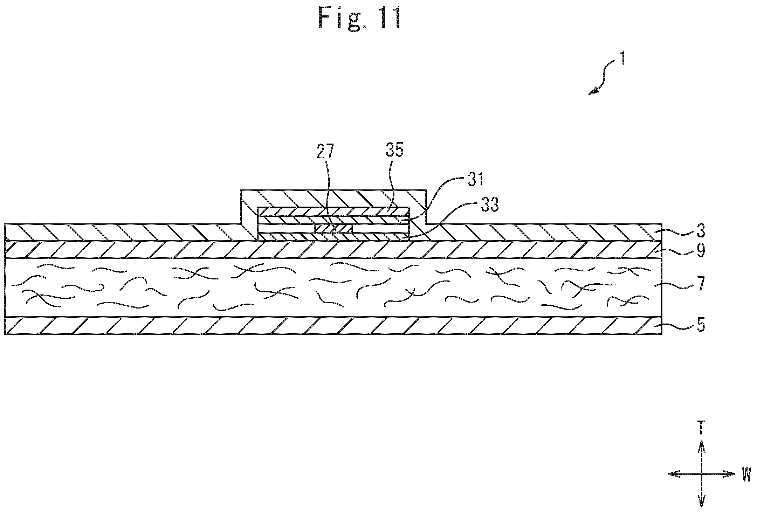

FIG. 11 is an end view of the III-III end face of FIG. 1 of the present invention B.

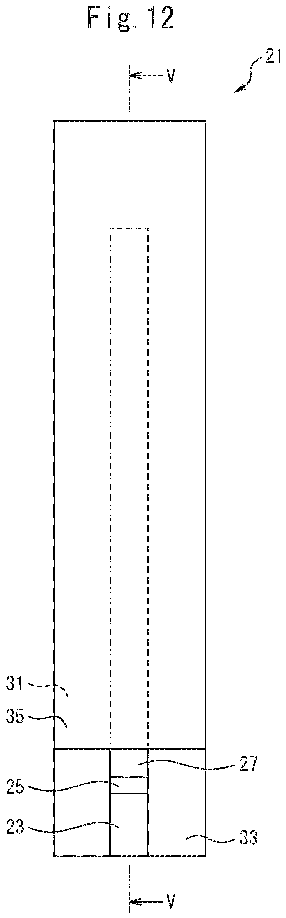

FIG. 12 is a plan view of the top sheet 3 side of a test strip 21 of the present invention B.

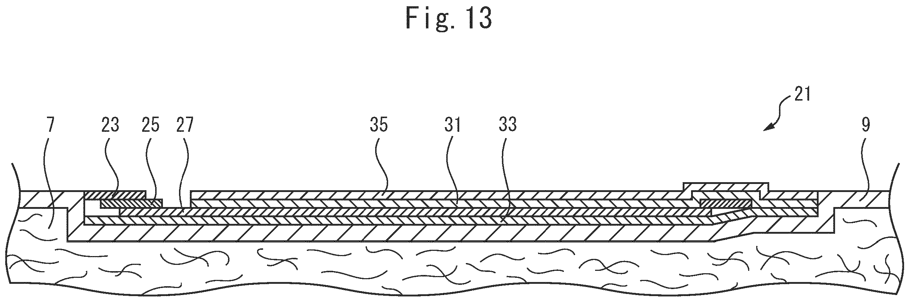

FIG. 13 is an end view of the V-V end face of FIG. 12 of the present invention B.

DESCRIPTION OF EMBODIMENTS

The details of the present invention will be described below.

<Aspect 1>

An absorbent article, comprising a test strip for detecting luteinizing hormone from vaginal discharge using immunochromatography, wherein
the test strip detects 0.05 mIU/mL to 2.5 mIU/mL of luteinizing hormone as positive.
As described above, in conventional methods for predicting the day of ovulation, urine is generally used as the detection target. Conversely, the present inventors have focused on vaginal discharge as a detection target used to predict the day of ovulation. Though there has been little knowledge regarding luteinizing hormone (LH) in vaginal discharge to date, according to the absorbent article comprising the present test strip for detecting 0.05 mIU/mL to 2.5 mIU/mL of luteinizing hormone as positive, luteinizing hormone in vaginal discharge can be reliably detected.
Furthermore, according to the present absorbent article, unlike conventional urinary luteinizing hormone (LH) testing methods, the day of ovulation can be predicted with high reliability while reducing the burden on users in that there is no need for conscious sampling of urine.
Furthermore, unlike conventional urinary luteinizing hormone (LH) test kits, the present absorbent article is an article for everyday use, making it possible to providing the effect of predicting the day of ovulation in a very convenient manner and with less psychological resistance.
Conventional immunochromatography can require operations such as applying urine to a test piece for detection, and take a relatively long time (for example, 10 minutes), whereas the present absorbent article allows the detection reaction to occur in conjunction with vaginal discharge simply by wearing the absorbent article, making it more convenient to predict the day of ovulation. According to the present absorbent article, for example, instead of having to wait 10 minutes to check the test results, detection results can be checked based on timing, such as when going to the bathroom, after 10 minutes have passed since the absorbent article was worn, which is thus convenient.
Furthermore, unlike conventional methods which use urine, the present absorbent article has an advantage in that detection can be performed without the concern of hand contamination.
As described above, in the invention according to Aspect 1 of the present application, an absorbent article which enables prediction of the day of ovulation in a more convenient manner than conventionally can be provided.
The LH concentration in urine can sometimes vary significantly depending on the amount of fluid intake, etc., whereas the LH concentration in vaginal discharge can be relatively stable. Thus, by using vaginal discharge as the detection target, effects which cannot be obtained using urine as the detection target can be obtained.

The test strip detects 0.05 mIU/mL to 5 mIU/mL, more preferably 0.05 mIU/mL to 2.5 mIU/mL (Aspect 3 of the present invention), and particularly preferably 0.05 mIU/mL to 2.0 mIU/mL, of luteinizing hormone as positive.

As will be easily understood by a person skilled in the art, "detects 0.05 mIU/mL to 2.5 mIU/mL of luteinizing hormone (LH) as positive" means that a positive result is indicated when the LH concentration is within this concentration range, but does not mean that a positive result is indicated "only" when the LH concentration is within the concentration range. Specifically, for example, a test strip which indicates a positive result for LH in the concentration range of 0.05 mIU/mL to 10.0 mIU/mL indicates a positive result for LH in the concentration range of 0.05 mIU/mL to 2.5 mIU/mL, and is thus included in the scope of the present invention.

An important point of the present invention is that, unlike conventional urinary LH detectors, the test strip is configured to detect LH within a relatively low concentration range of 0.05 mIU/mL to 2.5 mIU/mL as positive.

From the viewpoint of improving the reliability of detection, it is preferable that the test strip not detect as positive at concentrations in the range of less than 0.001 mIU/mL, and particularly preferably not detect as positive at concentrations in the range of less than 0.005 mIU/mL, less than 0.01 mIU/mL, less than 0.02 mIU/mL, less than 0.03 mIU/mL, less than 0.04 mIU/mL, or less than 0.05 mIU/mL.

The test strip according to Aspect 1 described above can detect as positive, for example, by color change that occurs when the luteinizing hormone is within the above concentration range.

Furthermore, in the test strip according to Aspect 1 described above, for example, when the luteinizing hormone is within the above-mentioned concentration range (for example, 0.05 mIU/mL to 2.5 mIU/mL), color change can occur at a level lower than the maximum level. The level of color change can be visually observed, for example.

A test strip capable of detecting LH within the above concentration range as positive can be produced by, for example, adjusting the sensitivity (for example, colorant) of a substance (for example, antibody) which reacts with luteinizing hormone, and in particular, by adjusting the size of a colorant adhering to the antibody which recognizes luteinizing hormone.

In particular, the test strip detects:

more preferably, 0.05 mIU/mL to 1.0 mIU/mL.
further preferably, 0.05 mIU/mL to 0.5 mIU/mL, and
even further preferably, 0.05 mIU/mL and 0.2 mIU/mL of luteinizing hormone as positive.

The detection concentration of the test strip can be measured using a casein blocking buffer solution of LH as a standard solution.

The casein blocking buffer solution of LH can be prepared, for example, in the following manner:

(a) A blocking buffer is produced by dissolving a casein blocking agent (1.0% casein, 100 mM borate, pH 8.5) in purified water.
(b) A standard LH solution of known concentration is diluted with the blocking buffer to produce an LH standard solution.

The casein blocking agent can be prepared, for example, as follows:
(1.0% Casein, 100 mM Borate, pH 8.5)

(a) Add 800 g of distilled water to a 1000 ml beaker.
(b) Add 2.0 ml of 20% NaOH.
(c) Stir for 10 minutes.
(d) Slowly add 10.0 g of casein powder.
(e) Stir for 3 hours.
(f) Add 6.18 g (= 100 mM) of boric acid.
(g) Stir for 10 minutes.
(h) Add 2.5 ml of 20% NaOH.
(i) Dilute to 1000 ml with distilled water.
(j) Stir for 10 minutes.
(k) Measure the pH.
(l) Filter using a filter unit (pore size 0.45 $\mu$m).
(m) Heat-treat at 30°C for 24 hours.
(n) Dispense into 15 ml or 50 ml containers for storage.
(o) Store at -80°C.

Conventional urinary LH testing methods are intended to detect a much higher concentration range as positive than the concentration range of 0.05 mIU/mL to 2.5 mIU/mL according to the present invention. Though it is possible to sample and detect LH in blood, this method is not as convenient as absorbent articles, since drawing blood is painful and causes bodily injury at the time of sampling.

For example, according to the prior art (Bioeng. Transl. Med., 2017, 2(3), pp. 238-246), the natural LH surge concentration in urine is 20 to 100 mIU/ml.

Conventional luteinizing hormone kits using urine specimen have a urinary luteinizing hormone concentration of 20 mIU/mL to 100 mIU/mL as a detection sensitivity range (Notification No. 0222-1, February 22, 2016, issued by the Director of Medical Device Evaluation Division, Pharmaceutical Safety and Environmental Health Bureau, Ministry of Health, Labor and Welfare).

The following literature relates to a study of reproductive hormone levels in urine during the menstrual cycle, and describes that the median total LH value was 38.3 (mIU/ml) on the day before ovulation (Table 2):
"Monitoring the Menstrual Cycle: Comparison of Urinary and Serum Reproductive Hormones Referenced True Ovulation", The European Journal of Contraception and Reproductive Health Care, 2015; Early Online: 1-13.

The following literature is a document on urinary and blood hormones, and describes the LH reference range during the ovulation phase of a woman as 8 to 100 (mIU/mL):
"Kansai Medical University - List of Endocrine Reference Values"

The following literature, which concerns urinary and blood hormones, lists a baseline LH value of 6.69 ($\pm$5.22) (mIU/mg Cr) and a peak LH value of 41.2 ($\pm$20.0) (mIU/mg Cr):
"Characteristics of Urinary Luteinizing Hormone Surge in Young Ovulatory Women" Fertil. Steril. 2007 Sep; 88(3); 684-90

<Aspect 1-2>

The present disclosure also includes:

An absorbent article, comprising a test strip for detecting luteinizing hormone from vaginal discharge using immunochromatography, wherein
the test strip detects 0.05 mIU/mL to 10 mIU/mL of luteinizing hormone as positive.

<Aspect 2>

In an aspect (Aspect 2) of the absorbent article according to the present disclosure, the test strip has a width of 5 mm or less.

The width of the test strip may be 1 mm to 10 mm, but is preferably 5 mm or less. The upper limit of the width of the test strip may further be 4 mm or less. In an embodiment of the present disclosure, the test strip has a width of 10 mm or less.

<Aspect 3>

In an aspect of the absorbent article according to the present disclosure, the test strip has a width of 3 mm or less.

From the viewpoints of visibility for the user and ease of processing, the width of the test strip is preferably 2 mm or more, and particularly preferably 2 to 3 mm.

In the absorbent article according to Aspects 2 and 3, since the width of the test strip is reduced to 5 mm or less, or further, 3 mm or less, the amount of liquid that needs to be retained for detection is relatively reduced. Thus, in the invention according to Aspect 2 or 3, even when the detection target is a vaginal discharge which is small in volume, has a high viscosity, and has a small amount of LH, LH can suitably be detected. Furthermore, when the width of the test strip is equal to or less than the above upper limit, the risk of damage to the labia during use of the absorbent article can be avoided.

Since the width of the test strip is reduced, there is an additional effect of reducing the discomfort experienced when wearing the absorbent article.

<Aspect 4>

In an aspect of the absorbent article according to the present disclosure, the test strip comprises:

a first member which contacts vaginal discharge,
a second member which contains a labeled antibody that recognizes luteinizing hormone in vaginal discharge, and
a third member which has a display part which contains a capture antibody,
the capture antibody is capable of capturing a complex formed by binding of the labeled antibody and luteinizing hormone, and

the test strip is configured such that the luteinizing hormone in vaginal discharge moves from the first member, through the second member, to the display part of the third member, and that

the display part of the third member changes color when the capture antibody captures the complex.

According to the absorbent article described in Aspect 4, luteinizing hormone can be detected in a convenient manner and quickly via a color reaction.

The measurement principle of the test strip will be described using a specific example. In an exemplary aspect, for example, the labeled antibody is a colorant-labeled mouse monoclonal antibody against human luteinizing hormone, and the capture antibody is a mouse monoclonal antibody against human luteinizing hormone. If human luteinizing hormone (hLH) is present in a specimen which has contacted the first member, the labeled antibody reacts with the hLH in the second member to form a complex based on the antigen-antibody reaction. This complex moves to the display part of the third member and is captured by the capture antibody immobilized and bound there, and as a result, the display part changes color based on the colorant (pigment).

A second display part (control part) can be provided downstream of the display part in the direction of antigen movement. A second capture antibody (for example, rabbit anti-mouse immunoglobulin polyclonal antibody) different from the capture antibody described above is immobilized and bound to this control part. This second capture antibody can recognize the labeled antibody. Labeled antibodies which do not form a complex with the antigen pass through the display part without being captured by the display part, and are captured by the control part, resulting in the control part changing color based on a colorant.

In this aspect, in the case of a positive result, the display part and the control part change color, and in the case of a negative result, only the control part changes color.

The configuration of the test strip will be described in detail later.

<Aspect 4-2>

In the absorbent article according to an embodiment of the present disclosure, the test strip comprises:

a second member containing a labeled antibody that recognizes luteinizing hormone in vaginal discharge, and
a third member which has a display part which contains a capture antibody,
the capture antibody is capable of capturing a complex formed by binding of the labeled antibody with the luteinizing hormone, and
the test strip is configured such that the luteinizing hormone in vaginal discharge moves from the second member to the display part of the third member, and that
the display part of the third member changes color when the capture antibody captures the complex.

According to the absorbent article described in Aspect 4-2, luteinizing hormone can be detected conveniently and quickly via a color reaction.

Though the manner in which vaginal discharge (or vaginal discharge components) is introduced into the test strip is not particularly limited, for example, it can be introduced by contacting the vaginal discharge with a portion of the test strip.

In an aspect of the absorbent article according to the present disclosure, the test strip comprises a first member which contacts vaginal discharge. In this case, the test strip may be configured such that luteinizing hormone in vaginal discharge moves from the first member, through the second member, to the display part of the third member.

<Aspect 5>

In the absorbent article according to an embodiment of the present disclosure, the first member, the second member, and the third member of the test strip are stacked in this order at least partially overlapping each other, and a sum of thicknesses of the first member, the second member, and the third member in the stacking direction is 1.5 mm or less.

As described above, vaginal discharge has a relatively high viscosity and low fluidity. Further, vaginal discharge is present in smaller amounts compared to urine. Thus, it has sometimes been difficult to detect LH in vaginal discharge by conventional methods, in particular, in detection using immunochromatography.

The present inventors have discovered that the amount of LH in vaginal discharge is smaller than that in urine. In conventional test strips, it has been difficult to detect LH in vaginal discharge.

In the absorbent article according to Aspect 5, since the thickness of the members constituting the test strip is reduced, the amount of liquid which needs to be retained in the test strip for detection is relatively reduced. Thus, in the absorbent article according to Aspect 5, even when the detection target is vaginal discharge, which is small in volume, has high viscosity, and contains a small amount of LH, LH can suitably be detected.

The thickness of each member (membrane thickness) is preferably in the range of 90 to 230 μm, and more preferably 100 to 220 μm. In particular, the thickness of each member (membrane thickness) is preferably in the range of 160 to 230 μm, and more preferably 180 to 220 μm.

<Aspect 6>

In an aspect of the absorbent article according to the present disclosure,

the first member is composed of fibers having an average fiber diameter of 0.5 to 5 μm (or a fineness of 0.01 to 0.1 dtex),
the second member is composed of fibers having an average fiber diameter of 10 to 40 μm (or a fineness of 2 to 6 dtex), and
an average fiber diameter of the fibers of the first member is less than an average fiber diameter of the fibers of the second member.

As described above, vaginal discharge has a relatively high viscosity and low fluidity. Thus, it has sometimes been difficult to detect LH in vaginal discharge, in particular, in detection using immunochromatography. Further, the present inventors discovered that the amount of LH in vaginal discharge is smaller than that in urine.

In the test strip according to Aspect 6, since the average fiber diameters of the members constituting the test strip are optimized, even vaginal discharge, which is present in a small amount and is highly viscous, is allowed to efficiently move to the detection area (display part), whereby detection can suitably be carried out.

The first member is preferably composed of fibers having an average fiber diameter of 1 to 4 μm, and more preferably an average fiber diameter of 1.5 to 3 μm.

The first member is preferably composed of fibers having a fineness of 0.02 to 0.08 dtex, and more preferably 0.03 to 0.06 dtex.

The second member is preferably composed of fibers having an average fiber diameter of 15 to 30 μm, and more preferably 18 to 25 μm.

The second member is preferably composed of fibers having a fineness of 2.5 to 5 dtex, and more preferably a fineness of 3 to 5 dtex.

The average fiber diameter of the fibers of the first member is preferably smaller than the average fiber diameter of the fibers of the second member, which can further improve the absorption by the first member, in particular, of relatively viscous vaginal discharge.

The average fiber diameters of the fibers constituting the first member and the second member can be measured by a scanning electron microscope, and the average fiber diameter can be the average value of the fiber diameters measured for N=30 or more fibers. Note that when measuring in a cross section, care should be taken because the shapes of the fibers may have changed when they were cut. Further, when measuring in a planar direction, since there may be cases in which the fibers have been fused, measurement should be performed avoiding portions where the shape has changed, such as fused parts.

<Aspect 7>

In an aspect of the absorbent article according to the present disclosure, an average fiber diameter R1 of the fibers constituting the first member and an average fiber diameter R2 of the fibers constituting the second member satisfy the relationship $5 \leq (R2/R1) \leq 20$.

According to this aspect, the relationship between the average fiber diameters of the first and second members constituting the test strip is optimized, and as a result, even vaginal discharge which is present in a small amount and is highly viscous is allowed to efficiently move to the detection area (display part), whereby detection can suitably be carried out. Specifically, the first member has improved absorbency, particularly for highly viscous vaginal discharge. Further, the effect of improved supporting rate of the labeled antibody in the second member is also obtained.

In a more preferable aspect, the following are satisfied:

$$6 \leq (R2/R1) \leq 18$$

$$7 \leq (R2/R1) \leq 16$$

$$8 \leq (R2/R1) \leq 14$$

$$9 \leq (R2/R1) \leq 12$$

<Aspect 7-2>

In an aspect of the absorbent article according to the present disclosure, a fineness D1 of the fibers constituting the first member and a fineness D2 of the fibers constituting the second member satisfy the relationship $50 \leq (D2/D1) \leq 200$.

According to this aspect, since the relationship between the finenesses of the first and second members constituting the test strip is optimized, even vaginal discharge which is present in a small amount and is highly viscous is allowed to efficiently move to the detection area (display part), whereby detection can suitably be carried out.

In a more preferable aspect, the following are satisfied:

$$60 \leq (D2/D1) \leq 190$$

$$70 \leq (D2/D1) \leq 180$$

$$80 \leq (D2/D1) \leq 170$$

$$90 \leq (D2/D1) \leq 160$$

<Aspect 8>

An absorbent article according to an embodiment of the present disclosure satisfies at least one of the following:

(a) the second member has a basis weight of 100 gsm or more,
(b) a sum of the thicknesses of the first member, the second member, and the third member is 1.5 mm or less, and the thickness of the second member accounts for 30% or more of this sum of the thicknesses, and
(c) the second member has a density of 0.05 to 0.80 $g/m^3$.

According to the invention described in Aspect 8, the basis weight, thickness, and/or density of the second member (conjugate pad) is optimized, thereby improving the diffusibility of the labeled antibody. Thus, the detection can suitably be performed even from vaginal discharge with a relatively high viscosity. Furthermore, within this range (in particular, the range of (c)), suitable liquid migration and color development are ensured.

<Aspect 9>

In an aspect of the absorbent article according to the present disclosure, the labeled antibody comprises a colorant having a particle diameter of 10 nm to 500 nm.

When the colorant of the labeled antibody which binds to LH has a particle diameter of 10 nm to 500 nm, sufficient detectability can be secured.

Examples of such colorants include colloidal gold and cellulose nanoparticles. Colloidal gold generally has a particle diameter of approximately 40 to 100 nm.

<Aspect 10>

In the absorbent article according to an embodiment of the present disclosure, the labeled antibody comprises a colorant having a particle diameter of 250 nm to 500 nm (preferably 280 to 450 nm, and more preferably 300 nm to 400 nm). Examples of such colorants include cellulose nanoparticles.

As described above, the present inventors have discovered that the amount of LH in vaginal discharge is smaller than that in urine. In this case, it is not easy to detect LH in vaginal discharge using conventional test strips (in particular, those targeting LH in urine).

In the test strip according to Aspect 10, the labeled antibody which binds to LH contains a colorant having a relatively large particle diameter, improving the visibility for detection. Thus, even when the detection target is vaginal discharge which contains a small amount of LH, the LH can suitably be detected.

<Aspect 10-2>

In an embodiment of the absorbent article according to the present disclosure, the test strip comprises a moving part which connects the first member and the display part, and has a first direction, which is a movement direction of vaginal discharge components from the moving part to the display part, a second direction orthogonal to the first direction, and a thickness direction orthogonal to the first direction and the second direction,

the test strip has, among five equal regions obtained by dividing the test strip in the second direction, a center region located at the center in the second direction and side regions located at both ends in the second direction, the moving part and the display part are arranged at least in the center region and the side regions, and a maximum thickness of the center region is greater than a maximum thickness of each side region, and/or an average thickness of the center region is greater than an average thickness of each side region.

In the test strip according to the absorbent article of this aspect, the center region has a greater average thickness than each side region, whereby a large volume of the space through which the discharge components move can be secured. Thus, the vaginal discharge components move easily in the first direction in the center region, whereby the color reaction can easily be shown in the display part in the center region. By the color reaction being shown at the center in the second direction of the test strip, the wearer can easily visually recognize the color reaction, whereby the visibility of the test result can be improved. In particular, the first direction and the second direction can be defined in the plane of the test strip, and in this case, the second direction is orthogonal to the first direction in the plane of the test strip. The five equal regions, and in particular, the center region and the side regions, may extend along the first direction.

<Aspect 10-3>

In an embodiment of the absorbent article according to the present disclosure, the display part of the test strip is visible from the skin side of the absorbent article,
the test strip comprises:

a migration-inhibiting upper layer which inhibits the migration of excretions of the wearer to the display part of the test strip, and
a migration-inhibiting lower layer which inhibits the migration of excretions of the wearer to the display part, and
the migration-inhibiting upper layer and the migration-inhibiting lower layer abut on an outside of the test strip in the width direction of the test strip.

In the absorbent article according to this aspect, since the test strip comprises a migration-inhibiting upper layer and a migration-inhibiting lower layer, and these layers abut on the outside of the test strip in the width direction of the test strip, unintended mixing of excretions (vaginal discharge) from the width direction and/or thickness direction of the test strip can be inhibited, and in particular, the migration of excretions (particularly vaginal discharge) directly to the display part without passing through the sample pad and conjugate pad of the test strip can be inhibited. As a result, this absorbent article can detect an antigen contained in vaginal discharge in a state in which the detection is less susceptible to the influence of vaginal discharge, etc.

The migration-inhibiting upper layer and the migration-inhibiting lower layer abutting on the outside of the test strip in the width direction of the test strip, more specifically, means that, for example, both ends of the migration-inhibiting upper layer and of the migration-inhibiting lower layer in the width direction extend beyond both ends of the test strip in the width direction and abut each other. Specifically, for example, when viewed from the skin side, the migration-inhibiting upper layer and the migration-inhibiting lower layer each have a width which is greater than the test strip and abut each other at both ends in the width direction.

When the test strip is arranged between a top sheet and the absorbent body, for example, the "migration-inhibiting lower layer" can inhibit the migration of excretions absorbed by the absorbent body to the display part.

In the absorbent article according to Aspect 10-3, since the test strip is visible from the skin side of the absorbent article, the position of the test strip of the absorbent article can be understood when worn, and the test strip can be arranged in an appropriate position. Further, since the test strip is visible from the skin side, by visually observing the absorbent article from the skin side of the absorbent article, the test result can be understood without peeling the absorbent article off the worn article or removing the absorbent article from the worn article.

The migration-inhibiting upper layer and the migration-inhibiting lower layer may each be a sheet, and for example, a sheet composed of a hydrophobic material, in particular, a liquid-impermeable sheet. This liquid-impermeable sheet has a lower breathability as compared to a sheet having an inter-fiber distance, such as a nonwoven fabric.

By interposing the test strip between two sheets serving respectively as the migration-inhibiting upper layer and the migration-inhibiting lower layer, the test target can be kept in place and the test strip can be prevented from drying out. Specifically, a suitable humidity can be maintained, and when the vaginal discharge and vaginal discharge components have been introduced, the vaginal discharge components are allowed to smoothly move, and the test can be appropriately performed.

Regarding the "migration-inhibiting upper layer" and the "migration-inhibiting lower layer", a seal part for sealing their outer edges is provided. In particular, the seal part is formed along both ends of the test strip in the width direction, whereby undesirable migration of excretions (particularly vaginal discharge) to the test strip is inhibited.

The "migration-inhibiting upper layer" and the "migration-inhibiting lower layer" are preferably configured so as to inhibit not only the migration of excretions of the wearer to the display part of the test strip but also the migration of excretions of the wearer to the moving part (for example, 66A in FIG. 1) of the test strip. For example, the "migration-inhibiting upper layer" and the "migration-inhibiting lower layer" may be arranged so as to overlap and cover at least the display part and the moving part of the test strip when viewed from the skin side.

<Aspect 11>

In an embodiment of the absorbent article according to the present disclosure, the absorbent article comprises a top sheet, and

during use of the absorbent article, the vaginal discharge reaches the test strip through the top sheet.

In the invention according to Aspect 11, discomfort experienced due to the test strip when wearing the absorbent article can be alleviated by the top sheet.

<Aspect 11-2>

In an embodiment of the absorbent article according to the present disclosure, the absorbent article has a front-rear direction, a width direction which is orthogonal to the front-rear direction, and a thickness direction which is orthogonal to the front-rear direction and the width direction,

in the thickness direction, starting from the skin side of a wearer in use, the top sheet and the absorbent body containing an absorbent material are further provided in this order, and
the test strip is arranged between the top sheet and the absorbent body.

According to this aspect, since the test strip is arranged between the top sheet and the absorbent body (absorbent core), the excreted vaginal discharge is allowed to efficiently migrate to the test strip (in particular, the first member of the test strip) during the vaginal discharge migrating to the absorbent article.

<Aspect 11-3>

In an embodiment of the absorbent article according to the present disclosure, the test strip comprises:

a second member containing a labeled antibody which recognizes luteinizing hormone in vaginal discharge, and
a third member which has a display part which contains a capture antibody,
the capture antibody is capable of capturing a complex formed by binding of the labeled antibody and luteinizing hormone,
the test strip is configured such that the luteinizing hormone in vaginal discharge moves from the second member to the display part of the third member, and that
the display part of the third member changes color when the capture antibody captures the complex,
a skin side sheet having a colored region is arranged closer to a skin side than the test strip,
the labeled antibody in the second member of the test strip contains a colorant, and
when viewed from the skin side, the labeled antibody in the second member is covered by the colored region of the skin side sheet.

According to this aspect, when viewed from the skin side, the colored labeled antibody in the second member (for example, the conjugate pad) of the test strip is covered by the colored region of the skin side sheet. According to this aspect, when the user visually observes the absorbent article before use from the skin side, the colored labeled antibody is difficult to visually recognize, thereby reducing the chance of the user misrecognizing that the reaction has already occurred in the test member before the absorbent article is used.

Further, since the labeled antibody in the second member (particularly, a reaction part) of the test strip is colored, not only the labeled antibody but also the complex stands out from the surroundings as compared to a configuration in which the labeled antibody is not colored. Thus, during the test, a region other than the color change portion of the display part is also colored, whereby progression of the test can be indicated. Furthermore, after the test is completed, the complex and the display part where a color reaction occurred due to the complex can be made to stand out. By allowing the display part where a color reaction occurred to stand out, the user can accurately and easily understand the test results, thereby reducing the chance of the user's misrecognition.

Though the manner in which vaginal discharge (or vaginal discharge components) is introduced into the test strip is not particularly limited, for example, it can be introduced by contacting the vaginal discharge with a portion of the test strip. In an embodiment, the test strip comprises a first member which contacts the vaginal discharge. In this case, the test strip can be configured such that the luteinizing hormone in vaginal discharge moves from the first member, through the second member, to the display part of the third member.

For more detailed specific aspects of the test strip according to Aspect 11-3, reference can be made to the above descriptions of the test strip.

<Aspect 12>

In an embodiment of the absorbent article according to the present disclosure, the detection is performed by directly contacting at least a portion of the top sheet with the vulva.

In the invention according to Aspect 12, since the vaginal discharge having adhered to the vulva can be collected in the absorbent article, the detection can efficiently be carried out even from vaginal discharge present in a relatively small amount.

In particular, the absorbent article is configured such that, when worn, the area (in particular, the first member) of the test strip to which the vaginal discharge adheres is arranged in an excretory opening contact area.

<Aspect 13>

In an embodiment of the absorbent article according to the present disclosure, the top sheet has a basis weight of 20 gsm or less.

In the invention according to Aspect 13, since the basis weight of the top sheet is reduced, vaginal discharge, which is a liquid substance having a high viscosity, can pass through the top sheet relatively easily and contact the test strip. Thus, even if the amount of vaginal discharge excreted is relatively small, detection can suitably be carried out.

In the invention according to Aspect 13, since the basis weight of the top sheet is relatively reduced, visibility of the test strip through the top sheet is secured.

Furthermore, in the invention according to Aspect 13, since the basis weight of the top sheet is relatively reduced, the top sheet is relatively soft. Thus, since conformability to the body of the wearer is improved, even if vaginal discharge adheres and remains on the body of the wearer, the vaginal discharge having adhered to the body can be transferred to the top sheet by movement of the wearer, etc. Thus, even if the amount of vaginal discharge excreted is relatively small, since it can be brought into contact with the test strip, detection can suitably be carried out.

<Aspect 14>

In an embodiment of the absorbent article according to the present disclosure, the top sheet has a thickness of 1 mm or less.

In the invention according to Aspect 14, since the thickness of the top sheet is reduced, the vaginal discharge, which is a liquid substance having a high viscosity, can pass through the top sheet relatively easily and reach the test strip. Thus, even if the amount of the vaginal discharge excreted is relatively small, detection can suitably be carried out.

In the invention according to Aspect 14, since the thickness of the top sheet is relatively reduced, visibility of the test strip through the top sheet is secured.

Furthermore, in the invention according to Aspect 14, since the thickness of the top sheet is relatively reduced, the top sheet is relatively soft. Thus, since conformability to the body of the wearer is improved, even if vaginal discharge adheres and remains on the body of the wearer, the vaginal discharge having adhered on the body can be transferred to the top sheet by movement of the wearer, etc. Thus, detection can suitably be carried out even from vaginal discharge present in a relatively small amount for sampling.

<Aspect 15>

In an embodiment of the absorbent article according to the present disclosure, the fibers constituting the top sheet have a single fiber diameter of 8.0 dtex or less.

In the invention according to Aspect 15, since the diameter of the fibers of the top sheet arranged between the wearer and the test strip is reduced, the gap in the top sheet is relatively large. Thus, the vaginal discharge, which is a liquid substance having a high viscosity, can pass through the top sheet relatively easily and reach the test strip. Thus, even if the amount of the vaginal discharge excreted is relatively small, detection can suitably be carried out.

Furthermore, in the invention according to Aspect 15, the top sheet is relatively soft because the diameter of the fibers of the top sheet is relatively reduced. Thus, since conformability to the body of the wearer is improved, even if vaginal discharge adheres and remains on the body of the wearer, the vaginal discharge having adhered to the body can be transferred to the top sheet by movement of the wearer, etc. Thus, detection can suitably be carried out even from vaginal discharge present in a relatively small amount for sampling.

The single fiber diameter of the fibers constituting the top sheet is more preferably 5.0 dtex or less, and further preferably 3.0 dtex. When the single fiber diameter of the fibers constituting the top sheet is 3.0 dtex or less, a particularly suitable skin feel can be obtained.

<Aspect 16>

In an aspect of the absorbent article according to the present disclosure,

the basis weight of the top sheet is 5 gsm or more,
the thickness of the top sheet is 0.1 mm or more, and
the single fiber diameter of the fibers constituting the top sheet is 1.0 dtex or more.

When vaginal discharge is brought into contact with the test strip through the top sheet, it is necessary to improve the permeability of the top sheet in order to bring the vaginal discharge, which has a relatively high viscosity, into suitable contact with the test strip.

Furthermore, when vaginal discharge is brought into contact with the test strip via the top sheet, it is necessary to ensure the visibility of the test strip as well as the wearability (texture) of the top sheet.

Furthermore, in order to efficiently collect vaginal discharge, which is excreted in a relatively small amount, and bring it into contact with the test strip, it is important that the top sheet be soft enough to transfer the vaginal discharge having adhered to the body of the wearer by their movements, etc., to the top sheet (transferability).

According to the invention described in Aspect 16, an absorbent article which satisfies all of the important requirements for effectively detecting LH from vaginal discharge (i.e., "liquid permeability", "visibility", "wearability", and "transferability") at a high level can be obtained.

<Aspect 17>

In an embodiment of the absorbent article according to the present disclosure,

the top sheet at least partially has an embossed pattern, and
a width of the test strip is greater than a size and/or spacing of each pattern of the embossed pattern.

When a top sheet is embossed, fibers constituting the top sheet become thicker due to the embossing, resulting in a decrease in the permeability of vaginal discharge and/or visibility through the top sheet in the area which has the embossed pattern.

In the invention according to Aspect 17, the width of the test strip is relatively large, whereby a decrease in permeability and/or visibility caused by the embossed pattern can be avoided.

<Aspect 18>

In an embodiment of the absorbent article according to the present disclosure,
the top sheet partially has an embossed pattern, and
the test strip is capable of contacting the vaginal discharge through an area of the top sheet which does not have the embossed pattern.

When a top sheet is embossed, fibers that constituting the top sheet become thicker due to the embossing, resulting in a decrease in the permeability of vaginal discharge and/or visibility through the top sheet in the area which has the embossed pattern.

In the invention according to Aspect 18, the width of the test strip is relatively large, whereby a decrease in permeability and/or visibility caused by the embossed pattern can be avoided.

<Aspect 19>

In an embodiment of the absorbent article according to the present disclosure,
the top sheet does not contain TiO2.

According to the invention described in Aspect 19, since the visibility of the top sheet is further improved, the visibility of the test strip through the top sheet can be further improved.

<Aspect 20>

The absorbent article according to any one of Aspects 11 to 19, wherein the test strip comprises a sample pad as a first member which contacts vaginal discharge, the sample pad is arranged in a position which faces a vaginal part of a wearer when worn, and
an average fiber diameter of fibers constituting the sample pad is equal to or less than an average fiber diameter of fibers constituting the top sheet.

According to the invention described in Aspect 20, solid matter which could not be filtered out by the top sheet can be filtered out by the sample pad, whereby LH in vaginal discharge can more easily be detected.

<Aspect 20-2>

In an embodiment, the test strip comprises a sample pad as a first member which contacts vaginal discharge, the sample pad is arranged in a position which faces a vaginal part of a wearer when worn,

the top sheet and the sample pad are composed of sheets containing fibers, and
an average inter-fiber distance of the top sheet is longer than an average inter-fiber distance of the sample pad.

According to this aspect, excretions (in particular, vaginal discharge) in the top sheet is more likely to migrate to the first member (in particular, the sample pad), which has a relatively narrow inter-fiber distance, whereby the detection can more easily be performed through the test strip even from vaginal discharge present in a small amount.

<Aspect 21>

A test strip for an absorbent article for detecting luteinizing hormone in vaginal discharge using immunochromatography, wherein
0.05 mIU/mL to 2.5 mIU/mL of luteinizing hormone is detected as positive.

In the present test strip, 0.05 mIU/mL to 2.5 mIU/mL of luteinizing hormone is detected as positive, whereby luteinizing hormone in vaginal discharge can more reliably be detected.

Furthermore, by using the present test strip in an absorbent article, the burden on the user can be reduced by eliminating the need for conscious sampling of urine while still enabling reliable prediction of the day of ovulation, unlike conventional urinary luteinizing hormone (LH) testing methods.

Furthermore, by using the present test strip in an absorbent article, the effect can be provided in that the day of ovulation can be predicted in a more convenient manner with less psychological resistance than in conventional test kits for urinary luteinizing hormone (LH).

Immunochromatography can require a relatively long time (for example, 10 minutes) for detection. By using the present test strip in an absorbent article, the detection reaction occurs in conjunction with vaginal discharge simply by wearing the absorbent article, making it more convenient to predict the day of ovulation.

Unlike conventional methods of using urine, by using the present test strip in an absorbent article, a further advantage is obtained in that detection can be performed without the concern of hand contamination.

As described above, according to Aspect 21 of the present application, a test strip which enables prediction of the day of ovulation in a more convenient manner than conventionally can be provided.

Regarding the details of the test strip according to Aspect 21, reference can be made to the above descriptions of the absorbent articles according to Aspects 1 to 20 (in particular, the descriptions of the test strip).

<Schematic Configuration of Absorbent Article>

The absorbent article 1 according to an embodiment will be described with reference to the drawings. The absorbent article may be an absorbent article such as a panty liner. In the following embodiment, a panty liner will be described as an example of an absorbent article. Note that the drawings are not necessarily to scale and are not intended to limit the present invention.

The absorbent article 1 of FIG. 3-1 may comprise an absorbent core 20, a top sheet 30, a back sheet, an adhesive part, and a test strip (test member) 60. The absorbent core 20 contains an absorbent material for absorbing liquid. The absorbent material may be composed of, for example, at least one of pulp and a superabsorbent polymer.

The absorbent article 1 of FIG. 3-1 may have a front-rear direction L, a width direction W, and a thickness direction T. The front-rear direction L is a direction extending from the front (ventral side) of the user to the rear (back side), or from the rear of the user to the front. The width direction W is a direction orthogonal to the front-rear direction L. The thickness direction T is a direction extending from the skin side of the user to the non-skin side, or from the non-skin side of the user to the skin side. The thickness direction T is a direction orthogonal to the front-rear direction L and the width direction W. The skin side corresponds to the side facing the skin of the user during use. The non-skin side corresponds to the side opposite to the skin side, i.e., the side facing away from the skin of the user during use.

As shown in FIG. 3-1, the absorbent article 1 may have a front section S1, a rear section S2, and a central section S3. The front section S1 is located in front relative to the central section S3, and the rear section S2 is located in rear relative to the central section S3. The central section S3 includes a region which contacts the excretory opening (for example, the vaginal opening) of the user. When dividing the absorbent article 1 into three equal parts in the front-rear direction L, the front region obtained may be the front section S1, the rear region obtained may be the rear section S2, and the central region between the front section S1 and the rear section S2 obtained may be the central section S3. When the absorbent article 1 has wings, the central section S3 may be a region from the front edge of the roots of the wings to the rear edge of the roots of the wings. Wings are portions which are folded back to the non-skin side of the worn article during use of the absorbent article 1.

The absorbent article 1 may comprise an absorbent core (absorbent body) 20, a top sheet 30 (particularly a surface sheet 31), a non-skin side sheet, an adhesive part, and a test strip (test member) 60. The absorbent core 20 contains an absorbent material for absorbing liquid. The absorbent material may be composed of, for example, at least one of pulp and a superabsorbent polymer.

The top sheet 30 is arranged closer to the skin side T1 than the absorbent body 20. The top sheet 30 may be a surface sheet 31 arranged closer to the skin side than the test strip 60. The surface sheet 31 faces the skin of the user. The surface sheet 31 may be composed of a plurality of sheets. In an embodiment, the top sheet 30 is composed of the surface sheet 31. The surface sheet 31 (top sheet 30) may be composed of, for example, a spunlace nonwoven fabric, an air-through nonwoven fabric, a spunbond nonwoven fabric, an SMS nonwoven fabric, etc.

The visible light transmittance of the surface sheet 31 may be 60% or more. The visible light transmittance of the surface sheet 31 may preferably be 70% or more, and more preferably 80% or more. The basis weight of the surface sheet 31 may be 90 gsm or less, 30 gsm or less, or 17 gsm or less. The thickness of the surface sheet 31 may be 5 mm or less.

The non-skin side sheet 40 is arranged closer to the non-skin side than the absorbent core 20. The non-skin side sheet 40 may comprise a back sheet 41 which faces the worn article. The non-skin side sheet 40 may be composed of, for example, a liquid-impermeable film. The non-skin side sheet 40 may also be a liquid-impermeable and breathable sheet, such as a perforated plastic film.

The adhesive part is provided on the non-skin surface of the non-skin side sheet 40. The adhesive part may be provided in a region overlapping the absorbent core 20 in the thickness direction T, or may be provided in a region not overlapping the absorbent core 20 in the thickness direction T. The adhesive part may be provided on the wings. The absorbent article 1 is affixed to the worn article via the adhesive part.

The configuration of the test strip will be described more specifically with reference to FIG. 3-1.

The test strip (test member) 60 of FIG. 3-1 has a display part (62A, 62B) which can change color based on excretions (vaginal discharge). The test strip 60 has a contact part (first member, for example, a sample pad) 64 which contacts the vaginal discharge. The test strip 60 may have a moving part through which components contained in the vaginal discharge (hereinafter, vaginal discharge components) move. In the moving part, the vaginal discharge components may move by capillary action. The moving part may have a display moving part 66A through which the vaginal discharge components move from the contact part 64 to the display part (62A, 62B), and a terminal moving part 66B through which the vaginal discharge components move in a direction away from the display part (62A, 62B). The terminal moving part is a part through which the vaginal discharge components that have passed through the display part (62A, 62B) move.

The contact part (first member) 64 with which the vaginal discharge comes into contact may be composed of, for example, a pad (which may be referred to as a sample pad or specimen pad). The vaginal discharge components in the vaginal discharge which have come into contact with the contact part 64 move, for example, through the pad and migrate to a second member (for example, a conjugate pad). The conjugate pad contains a labeled antibody which recognizes luteinizing hormone in vaginal discharge. Though the conjugate pad (second member) is not illustrated in FIG. 3-1, it may be arranged so as to overlap the sample pad (first member) in the thickness direction. The vaginal discharge components, the complex formed by the antigen-antibody reaction with the luteinizing hormone contained in the vaginal discharge components, and the labeled antibody may move through the moving part 66A by capillary action. The part where these substances move may be composed of, for example, a membrane. The display part has an area 62A containing a capture antibody for capturing (binding) the formed complex. This area changes color when the complex is captured by the capture antibody. This area 62A of the display part may be referred to as a test line.

The display part may have an area 62B containing a capture antibody in addition to the area 62A containing the capture antibody for capturing the complex. The capture antibody of the area 62B may capture a complex other than the complex captured in the area 62A, or may capture the labeled antibody. The area 62B changes color when a complex or the labeled antibody is captured. The area 62B for capturing the labeled antibody may be referred to as a control line. The vaginal discharge components which have passed through the display part 62 (i.e., the components which have not been captured by the capture antibody) move through the terminal moving part 66B. The terminal moving part 66B may be composed of a membrane. The terminal moving part 66B may also have an adsorption pad for absorbing the vaginal discharge components which have passed through the display part 62. The vaginal discharge components which have passed through the display part 62 may be absorbed by the absorbent core 20 instead of the adsorption pad. When immunochromatography is used, the display part 62 and the contact part 64 are arranged at different positions. Thus, in this case, the display part 62 and the contact part 64 are different.

The test strip (test member) 60 needs only to comprise members which are capable of retaining or containing substances such as labeled antibodies and captured antibodies, and may be composed of any of the materials such as paper, nonwoven fabric, or woven fabric.

The test strip (test member) 60 may be biased to one side in the front-rear direction L. As shown in FIG. 1, the center CT of the test member 60 in the front-rear direction L may be positioned in front relative to the center CL of the absorbent article 1 in the front-rear direction L. Furthermore, the display part (62A, 62B) may be biased to one side in the front-rear direction L. The display part (62A, 62B) may be positioned in front relative to the center CL of the absorbent article 1, and the display part (62A, 62B) may be positioned in front relative to the central section S3. The display part 62 may be arranged in the front section S1. As a result, the display part (62A, 62B) is arranged in a position distant from the excretory opening, whereby a reduction in visibility of the change in color of the display part (62A, 62B) caused by the vaginal discharge can be prevented. In particular, since vaginal discharge is less likely to diffuse to the front section S1 when the wear is in the supine position, a reduction in visibility of the change in color caused by the vaginal discharge can be prevented. Further, in the test member 60 using the immunochromatography, since the vaginal discharge is less likely to come into direct contact with the display part (62A, 62B), the health condition can be displayed with high accuracy.

The contact part 64 may be biased to one side in the front-rear direction L. The contact part 64 may be positioned in front relative to the center CL of the absorbent article 1. The contact part 64 may be arranged in a crotch section (not illustrated). Since the vaginal discharge is more likely to contact the contact part 64, the display part is more likely to change color, and the user can more easily check the health condition. The contact part 64 may be positioned more inward in the front-rear direction L than the display part (62A, 62B).

When a test strip region of the test strip 60 is divided into five equal parts in the width direction W, regarding the center region located in the center and two side regions located at both ends, the maximum thickness of the center region may be greater than the maximum thickness of each side region, and/or the average thickness of the center region may be greater than the average thickness of each side region.

A test strip (test member) is generally composed of a plurality of members each having a respective function, such as a moving part, a display part, etc., and if the respective constituent members become misaligned or curled, there is a risk of a reduction in test accuracy. When an absorbent article is worn, a test member is subjected to external forces due to movements, etc., of the wearer, and can become deformed, which may cause the members constituting the test member to become misaligned or curled.

Conversely, when the side regions each have a maximum thickness and/or average thickness less than the center region, the edges of the test member are less likely to become caught, curling at the edges of the test member can be inhibited, and test accuracy can be maintained.

By inhibiting curling and twisting at the edges of the test strip, local changes in the thickness and density of each constituent member can be inhibited. If the thickness and density change locally, the movement of excretion components may stagnate in a portion having undergone the change, or the color reaction may become difficult to occur. By inhibiting local changes in the thickness and density of each constituent member, a decrease in test accuracy can be inhibited.

A conceptual diagram of the test strip is shown in the attached FIG. 3-2. Regarding the other structures of the test strip, reference can be made to descriptions of, for example, WO 2021/132724A1.

FIG. 3-3 is a schematic cross-sectional view of an absorbent article according to an aspect of the present invention. A test strip (test member) is arranged between the top sheet and the absorbent core (absorbent body). In the illustrated aspect, a second sheet is arranged between the absorbent core and the test strip (test member).

FIG. 3-4 is a schematic cross-sectional view of an absorbent article according to an aspect of the present invention. The test strip comprises a sample pad, a conjugate pad, and a membrane (including a display part). When the absorbent article is viewed from the skin side, a colored region supported on a first skin side sheet covers the conjugate pad (second member). In this illustrated aspect, a pad positioned downstream of the membrane comprising the display part is also covered by the colored region.

<Method for Detecting Luteinizing Hormone from Vaginal Discharge>

The present disclosure includes a method for detecting luteinizing hormone from vaginal discharge using an absorbent article comprising a test strip using immunochromatography, the method comprising the step of:

allowing vaginal discharge to adhere to the test strip of the absorbent article, wherein
in the test strip, 0.05 mIU/mL to 2.5 mIU/mL of luteinizing hormone is detected as positive.

Regarding details of each constituent element in this method, such as the absorbent article and the test strip, reference can be made to the above descriptions of the test strip and the absorbent article.

The method of allowing vaginal discharge to adhere to the test strip of the absorbent article is not particularly limited, and reference can be made to the above descriptions regarding the absorbent article and the test strip. For example, the present worn article can be configured such that the vaginal discharge excreted from the wearer wearing the present absorbent article adheres to the test strip (in particular, the first member of the test strip) while worn. Furthermore, for example, the absorbent article comprises a top sheet and is configured such that the vaginal discharge reaches the test strip through the top sheet when the absorbent article is used, whereby the vaginal discharge can adhere to the test strip of the absorbent article. Preferably, at least a portion of the top sheet is in direct contact with the vulva.

In an embodiment of the method described above, 0.05 mIU/mL to 1.0 mIU/mL, or 0.05 mIU/mL to 0.5 mIU/mL, or 0.05 mIU/mL to 0.2 mIU/mL of luteinizing hormone is detected as positive.

<Absorbent Article for Estimating Day of Ovulation>

The present disclosure includes the following absorbent article ("Aspect X1"):

An absorbent article, comprising a test strip for detecting luteinizing hormone (LH) from vaginal discharge using immunochromatography, wherein
the test strip is configured to estimate that the wearer of the absorbent article is in a period spanning from 5 days before ovulation to the day of ovulation.

In the absorbent article according to Aspect X1, an absorbent article which enables a period suitable for conception attempts to be identified earlier than conventionally can be provided. In particular, according to this aspect, an absorbent article which enables a period suitable for conception attempts to be identified earlier than conventionally and with relatively high accuracy can be provided.

Furthermore, according to Aspect X1, an absorbent article which enables more convenient prediction of the day of ovulation than conventionally can be provided.

In conventional ovulation day prediction methods, and in particular, prediction methods based on urinary LH, there have been cases where the predicted ovulation day is so close that the timing of conception attempts does not align, resulting in missed opportunities. In the prediction of the ovulation day based on the menstrual cycle, there have been cases where the accuracy of identifying the ovulation day is low and sufficient outcomes in conception attempts cannot be achieved. In the present disclosure, "conception attempts" refers to sexual intercourse for the purpose of conceiving a child.

Furthermore, conventional ovulation day prediction methods using urine as a detection target have problems regarding convenience and the time and effort required to perform the detection operation and obtain results. Therefore, there have been cases where conventional ovulation day prediction methods are not useful for appropriately carrying out conception attempts.

Specifically, for example, the LH surge in urine peaks locally on the two days including the day of ovulation. By using the LH value in urine, it is possible to identify the two days including the day of ovulation. However, even if the user identifies the two days including the day of ovulation by testing the LH in urine, there have been cases where the day of ovulation is so close that it becomes impossible to plan conception attempts and thus even if the day of ovulation is identified, conception attempts are still not possible.

In order to facilitate the planning of conception attempts, it can be considered that the day of ovulation is anticipated based on the menstrual cycle. However, prediction of the day of ovulation based on the menstrual cycle has low accuracy in identifying the day of ovulation, and has not been able to achieve sufficient outcomes in conception attempts. Generally, it is said that menstruation occurs about 14 days from the day of ovulation, but this can vary depending on changes in physical condition, and it only provides a rough indication of the day of ovulation.

The absorbent article according to Aspect X1 comprises a test strip for detecting luteinizing hormone from vaginal discharge using immunochromatography, and this test strip is configured to estimate that the wearer of the absorbent article is in a period for conception attempts spanning from 5 days before ovulation to the day of ovulation. More specifically, the absorbent article according to Aspect X1 is configured to allow for the detection of luteinizing hormone (LH) in vaginal discharge excreted during the period for conception attempts spanning from 5 days before ovulation to the day of ovulation, for example, by changing color, whereby it can be determined with relatively high accuracy that the wearer is in a period for conception attempts spanning from 5 days before ovulation to the day of ovulation.

The invention of Aspect X1 is based on the new knowledge that by focusing on LH in vaginal discharge, the expected day of ovulation can be predicted earlier than conventionally. Specifically, the present inventors have discovered that by using LH in vaginal discharge as a detection target, it can be determined with a relatively high probability that the wearer of the absorbent article is in the period spanning from 5 days before ovulation to the day of ovulation (the period suitable for conception attempts).

According to this Aspect X1, since the day of ovulation can be identified from a relatively early stage as compared to the case in which LH surge in urine is used as an indicator, the possibility of securing more opportunities for conception attempts is increased, and conception attempts can be planned and scheduled. As compared to conventional aspects in which the day of ovulation can be identified on the day of or the day before ovulation, the burden of having to plan conception attempts on the day of, the day before, or two days before ovulation can be reduced, and/or situations where conception attempts cannot be made due to the inability to plan ahead can be reduced, which can result in increased ability to execute conception attempts while reducing mental stress.

Further, since the absorbent article according to Aspect X1 comprises a test strip for detecting luteinizing hormone from vaginal discharge using immunochromatography, the period for conception attempts can be identified with relatively high accuracy as compared to conventional techniques such as those using the menstrual cycle as an indicator.

Absorbent articles which absorb vaginal discharge are commonly used articles. Thus, in the absorbent article according to Aspect X1 of the present invention, the user can perform the test by wearing the present absorbent article in the same manner as an absorbent article which is normally worn.

Furthermore, since the test results of vaginal discharge are awaited while the absorbent article is worn, there is no need to set aside time solely for waiting for the results as is the case with urine-based testing, which reduces the mental and temporal burden of the test.

The attached FIGS. 8-1 to 8-3 are graphs showing LH surges in vaginal discharge. These graphs show that LH surges in vaginal discharge can be detected earlier than urinary LH surges. As is clear from these results, it is possible to identify a period suitable for conception (for example, the period spanning from 5 days before ovulation to the day of ovulation) by using the LH value in vaginal discharge as an indicator. The horizontal axes of FIGS. 8-1 to 8-3 indicate the number of days when the urinary LH peak is set to day 0.

The period for conception attempts to which the test strip of Aspect X1 can respond may be more limited, for example, a period for conception attempts spanning from 5 days before ovulation to the day before ovulation, a period for conception attempts spanning from 5 days before ovulation to 2 days before ovulation, a period for conception attempts spanning from 5 days before ovulation to 3 days before ovulation, or a period for conception attempts spanning from 5 days before ovulation to 4 days before ovulation.

When LH in vaginal discharge is set as the detection target, it is not necessarily possible to detect only the specific period for conception attempts described above. However, when LH in vaginal discharge is set as the detection target, since it is possible to predict the day of ovulation at a relatively earlier stage than conventionally, it is possible to determine that the wearer is in the above period for conception attempts with a higher probability than conventionally.

The detailed configuration of the absorbent article according to Aspect X1, i.e., the absorbent article comprising a test strip configured to estimate that the wearer of the absorbent article is in the period spanning from 5 days before ovulation to the day of ovulation, is not particularly limited, and can be realized using, for example, the test strip according to Aspect X2 below.

The present disclosure also includes the following absorbent article ("Aspect X2"):

The absorbent article according to Aspect X1, which is an absorbent article comprising a test strip for detecting luteinizing hormone from vaginal discharge using immunochromatography, wherein the test strip detects 0.05 mIU/mL to 10 mIU/mL of luteinizing hormone as positive.

According to the Aspect X2, a test strip for an absorbent article which enables a period suitable for conception attempts to be identified earlier than conventionally can be provided. In particular, according to this Aspect, a test strip for an absorbent article which enables a period suitable for conception attempts to be identified earlier with relatively higher accuracy than conventionally can be provided.

Furthermore, Aspect X2 can provide a test strip for an absorbent article which enables more convenient prediction of the day of ovulation than conventionally.

Without being bound by theory, since the amount of luteinizing hormone (LH) to be detected is relatively large in conventional urine test strips, the detection sensitivity is set relatively low accordingly. Conversely, in the invention according to Aspect X2 of the present invention, in order to detect luteinizing hormone in vaginal discharge present in a relatively small amount, the detection sensitivity is set relatively higher than that of conventional urine test strips. Thus, according to the absorbent article according to Aspect X2 of the present invention, the rise in progesterone at a relatively early stage, which could not be detected conventionally, can also be detected, and as a result, it is believed that a relatively early stage before the day of ovulation can be identified.

<Test Strip for Estimating Day of Ovulation>

The present disclosure also includes the following test strip ("Aspect Y1"):

A test strip for an absorbent article for detecting luteinizing hormone in vaginal discharge using immunochromatography, wherein
the test strip is configured to estimate that the wearer of the absorbent article is in a period spanning from 5 days before ovulation to the day of ovulation.

As described above with regard to Aspect X1, since the test strip of Aspect Y1 enables the day of ovulation to be identified from a relatively early stage, for example, as compared to the case where the LH surge in urine is used as an indicator, the possibility of securing more opportunities for conception attempts is increased, whereby conception attempts can be planned and scheduled. Further, as compared to conventional aspects in which the day of ovulation can be identified on the day of or the day before ovulation, the burden of having to plan conception attempts on the day of, the day before, or two days before ovulation can be reduced, and/or situations where conception attempts cannot be made due to the inability to plan ahead can be reduced, which can result in increased ability to execute conception attempts while reducing mental stress.

Furthermore, since the test strip of Aspect Y1 detects luteinizing hormone from vaginal discharge using immunochromatography, the period for conception attempts can be identified with higher accuracy as compared to conventional techniques, such as those using the menstrual cycle as an indicator.

Aspects related to Aspects X1, X2, and Y1 are shown below. Regarding the details of these Aspects, reference can be made to the above descriptions regarding Aspects 1 to 21 of the present invention:

<Aspect X1>

An absorbent article, comprising a test strip for detecting luteinizing hormone (LH) from vaginal discharge using immunochromatography, wherein
the test strip is configured to estimate that the wearer of the absorbent article is in a period spanning from 5 days before ovulation to the day of ovulation.

<Aspect X2>

The absorbent article according to Aspect X1, which is an absorbent article comprising a test strip for detecting luteinizing hormone from vaginal discharge using immunochromatography, wherein
the test strip detects 0.05 mIU/mL to 10 mIU/mL of luteinizing hormone as positive.

<Aspect X3>

An absorbent article, comprising a test strip for detecting luteinizing hormone from vaginal discharge using immunochromatography, wherein
the test strip detects 0.05 mIU/mL to 2.5 mIU/mL of luteinizing hormone as positive.

<Aspect X4>

The absorbent article according to any one of Aspect X1 to 3, wherein the test strip has a width of 10 mm or less.

<Aspect X5>

The absorbent article according to any one of Aspect X1 to 4, wherein the test strip comprises:

a second member which contains a labeled antibody that recognizes luteinizing hormone in vaginal discharge, and
a third member which has a display part which contains a capture antibody,
the capture antibody is capable of capturing a complex formed by binding of the labeled antibody and luteinizing hormone, and
the test strip is configured such that the luteinizing hormone in vaginal discharge moves from the second member to the display part of the third member, and that
the display part of the third member changes color when the capture antibody captures the complex.

<Aspect X6>

The absorbent article according to Aspect X5, wherein the labeled antibody comprises a colorant having a particle diameter of 250 nm to 500 nm,

<Aspect X7>

The test strip according to Aspect X5 or 6, wherein the test strip comprises a moving part for connecting the second member and the display part, and has a first direction, which is a movement direction of a vaginal discharge component from the moving part to the display part, a second direction orthogonal to the first direction, and a thickness direction orthogonal to the first direction and the second direction,

the test strip has a center region located at a center in the second direction and side regions located at both ends in the second direction, among regions obtained by dividing the test strip into five equal parts in the second direction,
the moving part and the display part are arranged at least in the center region and the side regions, and
a maximum thickness of the center region is greater than a maximum thickness of each of the side regions, and/or an average thickness of the center region is greater than an average thickness of each of the side regions.

<Aspect X8>

The absorbent article according to any one of Aspect X5 to 7, wherein the display part of the test strip is visible from a skin side of the absorbent article,
the test strip comprises:

a migration-inhibiting upper layer which inhibits migration of excretions of a wearer to the display part of the test strip, and
a migration-inhibiting lower layer which inhibits migration of excretions of the wearer to the display part, and
the migration-inhibiting upper layer and the migration-inhibiting lower layer abut on an outside of the test strip in the width direction of the test strip.

<Aspect X9>

The absorbent article according to any one of Aspects X1 to 8, wherein the absorbent article comprises a top sheet, and
during use of the absorbent article, the vaginal discharge reaches the test strip through the top sheet.

<Aspect X10>

The absorbent article according to Aspect X9, having a front-rear direction, a width direction orthogonal to the front-rear direction, and a thickness direction orthogonal to the front-rear direction and the width direction, wherein

in the thickness direction, starting from the skin side of a wearer during use, the top sheet and an absorbent body containing an absorbent material are further provided in this order, and
the test strip is arranged between the top sheet and the absorbent body.

<Aspect X11>

The absorbent article according to Aspect X9 or 10, wherein the test strip comprises the second member which contains a labeled antibody that recognizes luteinizing hormone in vaginal discharge, and the third member which has a display part which contains a capture antibody,

the capture antibody is capable of capturing a complex formed by binding of the labeled antibody and luteinizing hormone,

the test strip is configured such that the luteinizing hormone in vaginal discharge moves from the second member to the display part of the third member, and that
the display part of the third member changes color when the capture antibody captures the complex,
a skin-side sheet having a colored region is arranged closer to a skin side than the test strip,
the labeled antibody in the second member of the test strip comprises a colorant, and
when viewed from the skin side, the labeled antibody in the second member is covered by the colored region of the skin-side sheet.

<Aspect X12>

The absorbent article according to any one of Aspect X9 to 11, wherein the detection is performed by directly contacting at least a portion of the top sheet with a vagina.

<Aspect X13>

The absorbent article according to any one of Aspect X9 to 12, wherein the test strip comprises a sample pad as a first member for contacting vaginal discharge, and the sample pad is arranged in a position facing a vagina part of a wearer when the absorbent article is worn, and
a fiber diameter of fibers constituting the sample pad is less than or equal to a fiber diameter of fibers constituting the top sheet,

<Aspect X14>

The absorbent article according to any one of Aspect X9 to 13, wherein the test strip comprises a sample pad as a first member for contacting vaginal discharge, and the sample pad is arranged in a position facing a vagina part of a wearer when the absorbent article is worn,

the top sheet and the sample pad are composed of sheets containing fibers, and
an average inter-fiber distance of the top sheet is greater than an average inter-fiber distance of the sample pad.

<Aspect Y1>

A test strip for an absorbent article for detecting luteinizing hormone in vaginal discharge using immunochromatography, wherein
the test strip is configured to estimate that the wearer of the absorbent article is in a period spanning from 5 days before ovulation to the day of ovulation.

<Aspect Y2>

A test strip for an absorbent article for detecting luteinizing hormone in vaginal discharge using immunochromatography, wherein
0.05 mIU/mL to 2.5 mIU/mL of luteinizing hormone is detected as positive.

EXAMPLES

The present invention will be described in detail below with reference to the Examples, but the present invention is not limited to these Examples.

<Experimental Example>

<<Potential of Leukorrhea Luteinizing Hormone (LH) as Biomarker for Predicting Day of Ovulation>>

The use of leukorrhea luteinizing hormone (LH) as a biomarker for predicting the day of ovulation was investigated.

It is important for women who wish to become pregnant (women attempting to conceive) to identify the day of ovulation in advance. From the viewpoint of convenience, the most widely used test kit at the moment is a urinary luteinizing hormone (LH) test kit. Measurement of urinary LH is relatively convenient, as it can be completed in about 15 minutes after applying urine to the test kit. However, daily measurement is a heavy burden on the user, and the utilization rate of urinary LH test kits among women attempting to conceive is not high. Thus, in order to establish a new method of predicting the day of ovulation, we focused on leukorrhea as a measurement specimen. Not only can leukorrhea be sampled non-invasively, but it requires no conscious effort for sampling, making it a specimen that can achieve a "testing" with minimal user burden "where the measurement is completed before the user even realizes it". However, there are few research examples of leukorrhea as a measurement specimen, and the measurement of ovulation prediction biomarkers such as LH therein has not been reported. Thus, we detected leukorrheal LH and examined its potential as a biomarker.

The test methods and results as well as discussion and conclusion are shown below.

<Methods>

•Subject Recruitment

Subjects were women aged 20 years or older and under 40 years old who had no menstrual-related illnesses, and whether they had never been pregnant or had given birth was not an issue.

•Collection of Specimens from Subjects

Subjects were provided with urine specimen sampling kits and unscented panty liners, and urine and the panty liners from the 10th to 19th postmenstrual days were collected. To prevent evaporation of liquid from the panty liners, the panty liners were immediately packed in non-permeable films after removal and stored at -18°C or lower, and then transported at -15°C or lower and collected.

•Extraction of Leukorrhea Component from Panty Liners

The center portion of each of the collected panty liners was cut into a sizer of 2 cm$^2$, which was then immersed in 500 $\mu$l of PBS and centrifuged at 3600 rpm to collect the extract, which was used as a measurement specimen.

•Specimen Measurement

LH in urine and in leukorrhea was measured by Enzyme-Linked Immunosorbent Assay (ELISA). In order to exclude external factors influencing the concentration in the specimens, urine was measured based on urinary creatinine (ELISA) and leukorrhea was measured based on total protein amount (BCA assay), and the values were corrected.

•Definition of Leukorrhea Surge

The leukorrheal LH surge was defined as follows:

A concentration increase of at least 2-fold as compared to the previous day, the leukorrheal LH/total protein exceeding 0.05 mIU/mg, wherein
If multiple peaks are present, the earliest peak is used in place of the top peak, assuming an actual test for leukorrheal LH.

•Exclusion Criteria

Specimens which met any of the following conditions were excluded from measurement specimens:

Specimen in which no urinary LH surge appeared during the collection period;
Specimen in which the concentration increased during the collection period, possibly due to urinary LH surge, but the concentration did not decrease during the period; and
Specimen from which leukorrhea could not be collected and the day on which the leukorrheal LH surge started was unknown.

•Count of Specimen Used in Analysis

16 individuals
29 specimens

•Results

(1) FIGS. 4 and 5 are graphs plotting urinary LH concentrations and leukorrheal LH concentrations relative to the number of days based on the urinary LH surge. It is known that the urinary LH surge appears several hours later than the blood LH surge. The leukorrheal LH does not have a fixed relationship with the urinary LH surge. In addition to a pattern in which the leukorrheal LH surge and urinary LH surge were observed on the same day, there was also a pattern in which the leukorrheal LH surge was observed with a time shift before or after the urinary LH surge.

(2) Table 1 shows the day of ovulation anticipated from the urinary LH surge and the ratio of the numbers of specimens in which a leukorrheal LH surge was observed. For the fertile window, the criteria established by Dunson et al. (*2) was used. When the day of ovulation was determined based on the urinary LH surge, the ratio of the number of specimens in which a leukorrheal LH surge was observed appearing in the fertile window totaled 86%.

(*2) D. B. Dunson et al. Day-Specific Probabilities of Clinical Pregnancy Based on Two Studies with Perfect Measurements of Ovulation, Human Reproduction, 1999;14(7): pp. 1835-1839

[Table 1]

| Table 1: Number of days from day of ovulation predicted from urinary LH surge and Ratio of number of specimens in which leukorrheal LH surge was observed | | | |
|---|---|---|---|
| Number of days (Days) from day of ovulation predicted from urinary LH surge | Number of specimens in which leukorrheal LH surge was observed | Ratio | |
| -4 | 4 | 14% | Total 86% |
| -3 | 1 | 3% | |
| -2 | 8 | 28% | |
| -1 | 8 | 28% | |
| 0 (day of ovulation) | 4 | 14% | |
| Other than above | 4 | 14% | |
| Total | 29 | 100% | |

As can be seen from FIGS. 4 to 7, the LH surge in vaginal discharge appeared in the fertile window at a ratio of 86%. This indicates that the absorbent article according to the present invention can adequately function as a biomarker for predicting the day of ovulation.

(3) The surge intensity was measured by comparing the day of the surge with the day before surge. As a result, the leukorrheal LH showed a sensitivity of 85% and a specificity of 77% at a threshold of 0.15 mIU/mg. Furthermore, ROC analysis indicated that the AUC was 0.79. FIG. 6 is a graph showing the LH concentrations around the day of the leukorrheal LH surge. FIG. 7 is a graph showing the results of ROC analysis.

Discussion & Conclusion

Since the leukorrheal LH surge appeared in the fertile window at a ratio of 86%, it can adequately function as a biomarker. The leukorrheal LH surge was significantly intense and demonstrated a distinguishing capability with a sensitivity of 85% and a specificity of 77% when an appropriate measurement system was constructed.

This study shows the biological potential of a wearable system for measuring leukorrheal LH, and it is believed that the development of a testing device will increase the chances of women attempting to conceive being able to become pregnant with reduced burden.

<Summary of Results>

It is important for women hoping to become pregnant to identify the day of ovulation in advance. Currently, there are various methods for predicting the day of ovulation, such as direct observation using ultrasound, basal body temperature, and fern crystal observation, but the most widely used method is a test for luteinizing hormone (LH) in urine from the viewpoint of convenience, etc. Though the measurement of urinary LH is relatively convenient, as it can be completed in about 15 minutes after applying urine to a test kit, daily measurement is still a heavy burden on the user, and the utilization rate of urinary LH test kits among women attempting to conceive is not very high. Thus, our research group focused on leukorrhea as a specimen. Not only can leukorrhea be sampled non-invasively, but it has an advantage of minimizing the burden on the user, as conscious sampling thereof is not necessary. However, leukorrheal LH has not been thoroughly studied, and it was unclear how much LH is contained in leukorrhea and whether its behavior is sufficient to predict the day of ovulation. In this test, as described above, urine and leukorrhea from 29 subjects over 10 day-period, from 10 to 19 days after the start of menstruation were collected, and LH in urine and in leukorrhea of the subjects was measured and compared by ELISA. As a result, an LH surge similar to that in urine was observed in leukorrhea, and assuming that the urinary LH surge identified by the urinary LH test kit is 1 day before ovulation, the ratio of leukorrheal LH surge appearing "5 days before ovulation to 0 days before ovulation", when the probability of pregnancy is high, was 86% (Table 1 above). Furthermore, it was discovered that the amount of leukorrheal LH was less than that of urinary LH, and thus a detection system with higher sensitivity than that of commercially available urinary LH test kits is required for detection. Though the results of this test do not necessarily indicate that urinary LH and leukorrheal LH behave in the same manner in terms of various characteristics such as concentration and surge day, it is considered that it is fully possible to predict the day of ovulation by measuring leukorrheal LH.

<Examples>

As test strips according to the present invention, "Example 1" and "Example 2" were produced and their performances were examined.

(Standard Solution)

The LH standard solution was a casein blocking buffer solution of LH. Specifically, the positive specimen solution was "Lumipulse Presto LH LH Calibrator (250 mIU)" (Fujirebio Inc.: Code No. 291573), and the dilution solution was 1% casein, 100 mM borate, pH 8.5. The positive specimen solution was diluted with the dilution solution to an arbitrary concentration to prepare the standard solution.

(Example 1 (Spec 1))

The test strip of Example 1 comprised:

a first member which contacted vaginal discharge,
a second member which contained a labeled antibody that recognized luteinizing hormone in vaginal discharge, and
a third member which had a display part which contained a capture antibody,
the capture antibody was capable of capturing a complex formed by binding of the labeled antibody and luteinizing hormone, and
the test strip was configured such that the luteinizing hormone in vaginal discharge moved from the first member, through the second member, to the display part of the third member, and that
the display part of the third member changed color when the capture antibody captured the complex.

The test strip of Example 1 had a length of 12 mm and a width of 3 mm. The sample pad as the first member had a thickness of 0.1 mm, the conjugate pad as the second member had a thickness of 0.55 mm, and the membrane as the third member had a thickness of 0.1 mm, with the sum of the thicknesses of the first to third members being 0.75 mm. The average fiber diameter of the fibers constituting the first member was approximately 2 $\mu$m, and the average fiber diameter of the second member was approximately 20 $\mu$m. The labeled antibody in the conjugate pad contained red nanobeads having a particle diameter of 335 nm (product name: NanoAct, manufactured by Asahi Kasei Corporation) as a colorant.
The average fiber diameters of the fibers constituting the first member and the second member were obtained by measuring the fiber diameters of a sample cut to a predetermined size and set it in a scanning electron microscope (FlexSEM1000, manufactured by Hitachi High-Tech Corporation), and averaging the measured fiber diameter values of N=30 or more.

(Example 2 (Spec 2))

The overall configuration and dimensions of the test strip of Example 2 and the dimensions of the first to third members were the same as those of Example 1. The labeled antibody in the conjugate pad of Example 2 contained blue nanobeads having a particle diameter of 320 nm (product name: NanoAct, manufactured by Asahi Kasei Corporation) as a colorant.

Furthermore, the labeled antibodies used in Examples 1 and 2 differed in the manner in which they recognized LH. LH is a glycoprotein composed of two subunits $\alpha$ and $\beta$, and these subunits separate after the LH surge. The antibody used in Example 1 was capable of binding only to the assembly where the $\alpha$ subunit and $\beta$ subunit were bound together ($\alpha$-$\beta$), whereas the antibody used in Example 2 was capable of binding not only to the $\alpha$-$\beta$ assembly where the $\alpha$ subunit and $\beta$ subunit were bound together ($\alpha$-$\beta$) but also to the $\beta$ subunit.

Furthermore, in Example 2, as compared to Example 1, the color of the colorant was changed to blue, which was easily visible even if it was faint, and the amount of labeled antibody was doubled.

(Sensitivity Test)

Standard solutions containing various concentrations of LH were each dropped twice (each 24 $\mu$L) onto the sample pads of the test strips of Example 1 and Example 2, and the color change at the display parts was observed. The difference of Example 2 from Example 1 was that the detection accuracy was improved and the visibility was also improved, and thus the probability that the wearer could judge by themselves was improved.

The results are shown in FIG. 1.

As can be seen from FIG. 1, in Example 1, when the LH concentration was 0.5 or 1.0 mIU/mL or more, a color change at the display part was observed.

Regarding Example 1, a proportional relationship between the LH concentration and the color strength at least in the concentration range of 0.5 to 1 mIU/mL was observed.

Furthermore, as can be understood from FIG. 1, in Example 2, when the LH concentration was 0.05 or 0.1 mIU/mL or more, a color change at the display part was observed.

In Example 2, a proportional relationship between the LH concentration and the color strength at least in the concentration range of 0.05 to 1.0 mIU/mL was observed.

<Comparison>

The test strips of Examples 1 and 2 were compared in terms of color intensity.

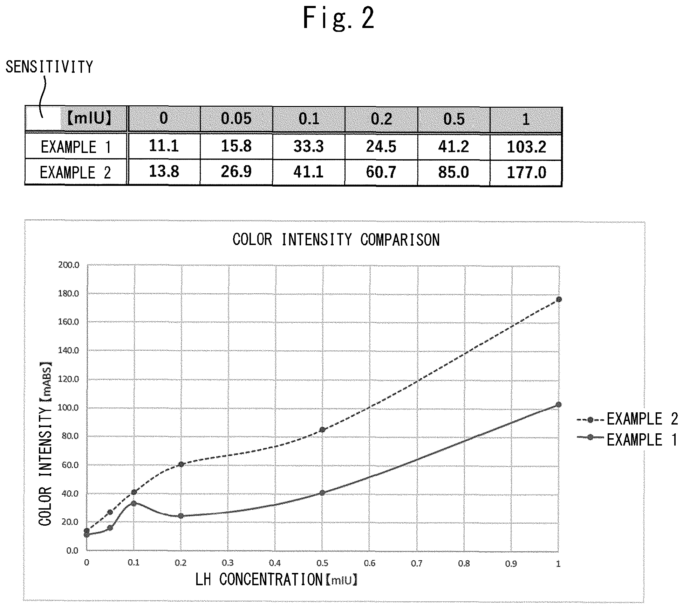

The results are shown in FIG. 2. As can be seen from FIG. 2, the test strip according to the present invention showed good color intensity over a wide range of LH concentrations. The strip of Example 2 showed better color intensity than the strip of Example 1.

<Vaginal Discharge Test>

Using panty liners comprising the test strips of the Examples 1 and 22 above, tests for detecting LH in vaginal discharge excreted from the vagina of the wearers were carried out.

The test results of Example 1 are shown in Table 2 below.

[Table 2]

Table 2

| Number of participants | 30 |
|---|---|
| LH surge was observed | 26 |
| Vaginal discharge adhered to LN | 25 |
| Vaginal discharge adhered to tester | 25 |
| Control line developed for 2 days | 21 |
| Test line developed | 15 |
| Test line was recognized | 5 |

As can be seen from Table 2, LH was detected with relatively high accuracy when the test strip of Example 1 was used. Specifically, among the 25 participants whose vaginal discharge adhered to the tester, the test line developed for 15 users (60%), and among them, the test line was recognized by 5 users (20%).

The test results of Example 2 are shown in Table 3 below.

[Table 3]

Table 3

| Number of participants | 28 |
| --- | --- |
| LH surge was observed | 26 |
| LH surge was observed, and vaginal discharge adhered to LN | 23 |
| LH surge was observed, and vaginal discharge adhered to tester | 23 |
| Control line developed for 2 days | 22 |
| Test line developed (researcher observed) | 22 |
| Test line was recognized by participant | 16 |

As can be seen from Table 3, LH was detected with relatively high accuracy when the test strip of Example 2 was used as well. In particular, the detection accuracy of Example 2 was higher than that of Example 1. Specifically, among the 23 participants whose vaginal discharge adhered to the tester, the test line developed for 22 users (94%), and among them, the test line was recognized by 16 users (72%).

<<Example 3 and Comparative Example 1>>

In Example 3 and Comparative Example 1, the test strip according to the present invention was applied to an absorbent article, and its performance was evaluated.

<Example 3>

(Manufacture of Absorbent Article Comprising Test Strip)

A test strip which detected 0.05 mIU/mL to 10.0 mIU/mL of LH as positive was prepared. This test strip had the same configuration as the test strip used in Example 2.
This test strip was applied to a panty liner (product name: Sofy Kiyora Unscented, manufactured by Unicharm Corporation) as an absorbent article to produce an absorbent article according to Example 3. Specifically, the test strip was arranged so that a sample pad portion of the test strip was arranged on the vaginal excretory opening contact portion of the top sheet constituting the panty liner. The test strip was affixed onto the top sheet with cover tape.

(Wearing Test)

A wearing test was carried out using the absorbent article of Example 3. Specifically, the absorbent article was worn in a general manner, and when vaginal discharge occurred, adhesion of the vaginal discharge to the test strip, reaction at the test line (presence or absence of a positive result), and the number of days relative to the day of ovulation were determined.
The wearing test was repeated to observe the reaction at the test line at various days relative to the day of ovulation.
The reaction at the test line (presence or absence of a positive result) was judged based on the presence or absence of color change, and the result was judged to be positive when color change was visually observed.
The number of days relative to the day of ovulation was determined with the day of ovulation predicted from the urinary LH surge defined as the reference day (day 0). The urinary LH surge was determined by measuring urinary creatinine (ELISA method) in urine sampled from the subjects.
The results are shown in Table 4 below.

<Comparative Example 1>

In Comparative Example 1, an absorbent article was produced in the same manner as in Example 3, urine was applied to a test strip on the absorbent article, and the reaction at the test line (presence or absence of a positive result) and the number of days relative to the day of ovulation were determined.

The adhesion of urine to the test strip was performed by dropping an appropriate amount of urine sampled from the subject onto the test strip with a dropper.

The observation of the reaction at the test line and the determination of the number of days relative to the day of ovulation were carried out in the same manner as in Example 3.

The results are shown in Table 4 below.

[Table 4]

Table 4

|  | Detection target | Presence/absence of adhesion of vaginal discharge to tester | Presence/absence of reaction at test line at day number before/after day of ovulation (numbers represent number of days relative to day of ovulation (0)) | | | | | | | | | | |
|---|---|---|---|---|---|---|---|---|---|---|---|---|---|
|  |  |  | -8 | -6 | -5 | -4 | -3 | -2 | -1 | 0 | 1 | 3 | 5 |
| Ex 3 | Vaginal discharge | Present | - | Absent | Present | Present | Present | Present | Present | Present | Absent | - | - |
| Comp Ex 1 | Urine | Present | Present | - | - | - | - | - | Present | - | - | Present | Present |

As can be seen in Table 4 above, a positive result at the test line was observed when an absorbent article (panty liner) comprising a test strip according to the present disclosure was worn and vaginal discharge excreted during the period from 5 days before ovulation (day -5) to the day of ovulation (day 0) adhered to the test strip.

Conversely, when vaginal discharge excreted one day after ovulation (day +1) or six days before ovulation (day -6) adhered to the test strip, no positive result at the test line was observed.

This result shows that the test strip according to the present invention can be used to predict the day of ovulation of the subject. By applying the test strip to the absorbent article, the day of ovulation can be predicted in a convenient manner. Specifically, it is sufficient that the subject wears the absorbent article as usual without performing any special operation, and when vaginal discharge occurs, the reaction at the test line on the test strip (presence or absence of color change, etc.) is observed.

Comparative Example 1 shows the results when urine was dropped onto the test strip of the present invention. As can be seen from Table 4, in Comparative Example 1, when urine sampled on days -8, -1, 3, and 5 from the day of ovulation was applied to the test strip, a positive result at the test line was observed in all the cases, regardless of the number of days from the day of ovulation.

Without intending to be bound by theory, this result indicates that the test strip of the present invention is optimized for detecting LH in vaginal discharge. Specifically, since the test strip of the present invention has a relatively high detection sensitivity for detecting LH in vaginal discharge, it is considered that when urine is applied to the test strip of the present invention, a positive reaction occurs regardless of the urinary LH surge due to the relatively high LH concentration in urine.

Though there is a risk of urine adhering to the test strip due to urinary leakage, etc. while wearing a panty liner, the risk of urine adhering to the test strip under normal wearing conditions or normal use is low.

<<Invention B>>

The present disclosure also includes the following invention ("the present invention B" or "invention B").

Invention B relates to an absorbent article.

<Background Related to Invention B>

Antigens are detected using bodily fluids such as urine and saliva to perform pregnancy tests, diagnose viral infections, and diagnose bacterial infections.

For example, Patent Literature (Japanese Unexamined Patent Publication (Kokai) No. 5-87807) discloses a pregnancy test device comprising a test device body comprising a urine sampling part which absorbs urine by contact, a reagent part which is arranged to contact the urine sampling part, a base part where urine sampled by the urine sampling part moves through the reagent part, a determination part which determines the presence or absence of pregnancy based on the presence or absence of color change caused by the urine that has moved through the base part, and a fixing member which integrally affixes the urine sampling part, the reagent part, the base part, and the determination part; and a bag which contains the test device body, wherein the bag comprises means for opening so that only the urine sampling part and the determination part of the test device body are sequentially exposed.

< Technical Problem of Invention B>

In general pregnancy test drugs, including the pregnancy test device disclosed in the Patent Literature (Japanese Unexamined Patent Publication (Kokai) No. 5-87807), human chorionic gonadotropin (hCG), an antigen contained in urine, is measured to determine the presence or absence of pregnancy. By using urine, the amount of the antigen can be measured in a convenient manner.

It is generally known that the concentration of components in urine decreases as the amount of fluid intake increases, and decreases when the amount of fluid intake decreases or when sweating increases. Thus, the concentration of antigens in urine is also likely to change. Therefore, in the case of pregnancy tests using urine, the measurement is recommended in the morning, when the concentration of hCCG in urine is high.

In general, it is considered that the amount of antigen in vaginal excretions is less affected by factors such as the amount of fluid intake than in urine. Therefore, if the antigen in vaginal excretions can easily be detected, the accuracy of antigen detection is thought to be improved. Furthermore, if the antigen in vaginal excretions can be easily detected, it is considered to be meaningful because it expands the options for detecting antigens. Furthermore, if the antigen contained in vaginal excretions is also contained in urine, it is expected that the accuracy of antigen detection will be improved by detecting both the antigen in vaginal excretions and the antigen in urine.

However, since vaginal excretions are generally not controllable by the will of the person excreting it, it tends to take time to detect an antigen contained in vaginal excretions.

Furthermore, since the vaginal opening and the urinary excretion opening are located close to each other, detection of antigens contained in vaginal excretions tends to be easily affected by urine.

Thus, an object of the present disclosure is to provide an absorbent article with which an antigen contained in vaginal excretions can be detected in a state in which the detection is less affected by urine.

< Solution to Problem of Invention B>

The present inventors have discovered the following Invention B (Aspects B1 to B14):

<Aspect B1>

An absorbent article, comprising a test strip for detecting an antigen contained in vaginal excretions and having a thickness direction, wherein

the absorbent article comprises a liquid-permeable top sheet, the test strip, an absorbent body, and a liquid-impermeable back sheet in that order in the thickness direction,

the test strip comprises, a sample pad for preventing mucin contained in the vaginal excretions from inhibiting an antigen-antibody reaction, a conjugate pad containing a labeled antibody, and a deployment membrane containing an immobilized antibody in that order in the thickness direction, and

the sample pad or the conjugate pad is in direct contact with the absorbent body, or indirectly in contact with the absorbent body via a liquid-permeable sheet.

<Aspect B2>

The absorbent article according to claim 1, wherein the antigen contained in the vaginal excretions is also contained in urine.

<Aspect B3>

The absorbent article according to Aspect B1 or B2, wherein the test strip further comprises, between the deployment layer and the absorbent body, a migration-inhibiting lower layer for inhibiting migration of excretions of a wearer absorbed by the absorbent body to the deployment layer.

<Aspect B4>

The absorbent article according to any one of Aspects B1 to B3, wherein the test strip further comprises, between the deployment layer and the top sheet, a migration-inhibiting upper layer for inhibiting migration of excretions of the wearer to the deployment layer.

<Aspect B5>

The absorbent article according to any one of Aspects B1 to B4, wherein the test strip further comprises, between the deployment layer and the top sheet, a migration-inhibiting upper layer for inhibiting migration of excretions of a wearer to the deployment layer, and the test strip further comprises, between the deployment layer and the absorbent body, a migration-inhibiting lower layer for inhibiting migration of excretions absorbed by the absorbent body to the deployment layer, and

the test strip further comprises a seal part for sealing outer edges of the migration-inhibiting upper layer and the migration-inhibiting lower layer.

<Aspect B6>

The absorbent article according to any one of Aspects B1 to B5, wherein a fiber density of the sample pad is greater than a fiber density of the top sheet.

<Aspect B7>

The absorbent article according to any one of Aspects B1 to B6, wherein a hydrophilicity of the sample pad is greater than a hydrophilicity of the top sheet.

<Aspect B8>

The absorbent article according to any one of Aspects B1 to B7, wherein the sample pad is in contact with the absorbent body, and a fiber density of the absorbent body is greater than a fiber density of the sample pad.

<Aspect B9>

The absorbent article according to any one of Aspects B1 to B8, wherein the sample pad is in contact with the absorbent body, and a hydrophilicity of the absorbent body is greater than a hydrophilicity of the sample pad.

<Aspect B10>

The absorbent article according to any one of Aspect B1 to B9, wherein a fiber density of the conjugate pad is greater than a fiber density of the sample pad.

<Aspect B11>

The absorbent article according to any one of Aspects B1 to B10, wherein a hydrophilicity of the conjugate pad is greater than a hydrophilicity of the sample pad.

<Aspect B12>

The absorbent article according to any one of Aspects B1 to B11, wherein the deployment layer is not in direct contact with the absorbent body.

<Aspect B13>

An absorbent article, comprising a test strip for detecting an antigen contained in vaginal excretions and having a thickness direction, wherein

the absorbent article comprises a liquid-permeable top sheet, the test strip, an absorbent body, and a liquid-impermeable back sheet in that order in the thickness direction, and
the test strip further comprises, between the test strip and the absorbent body, a migration-inhibiting lower layer for inhibiting migration of excretions of a wearer absorbed by the absorbent body to the test strip.

<Aspect B14>

The absorbent article according to Aspect B13, wherein the test strip further comprises, between the test strip and the top sheet, a migration-inhibiting upper layer for inhibiting migration of excretions other than vaginal excretions of a wearer to the test strip.

<Effects of Invention B>

The absorbent article according to Invention B is capable of detecting antigens contained in vaginal excretions in a state in which the detection is less susceptible to the influence of urine.

< Description of Embodiments of Invention B>

Specifically, the present invention B relates to the following aspects:

[Aspect B1]

An absorbent article, comprising a test strip for detecting an antigen contained in vaginal excretions and having a thickness direction, wherein

the absorbent article comprises a liquid-permeable top sheet, the test strip, an absorbent body, and a liquid-impermeable back sheet in that order in the thickness direction,
the test strip comprises, a sample pad for preventing mucin contained in the vaginal excretions from inhibiting an antigen-antibody reaction, a conjugate pad containing a labeled antibody, and a deployment membrane containing an immobilized antibody in that order in the thickness direction, and

the sample pad or the conjugate pad is in direct contact with the absorbent body, or indirectly in contact with the absorbent body via a liquid-permeable sheet.

In the absorbent article, vaginal excretions (for example, vaginal discharge or menstrual blood) reach the liquid-permeable sheet, then permeates the liquid-permeable sheet and reach the sample pad of the test strip. In the sample pad, the vaginal excretions are treated to prevent the mucin contained in the vaginal excretions from inhibiting the antigen-antibody reaction, and the vaginal excretions are then transferred to the conjugate pad. In the conjugate pad, the antigen contained in the vaginal excretions binds with the labeled antibody in the conjugate pad to form an immune complex, and the immune complex (vaginal excretions?) is transferred to the deployment membrane. In the deployment membrane, the immune complex binds with the immobilized antibody, causing color development, thereby detecting the antigen.

Since the vaginal opening and the urinary excretion opening are located close to each other, an absorbent article may absorb urine of the wearer, and the urine absorbed by an absorbent article may reach a test strip and adversely affect the detection of antigens.

In the absorbent article, since the sample pad or conjugate pad is in direct contact with the absorbent body or indirectly in contact with the absorbent body via a liquid-permeable sheet, urine which has reached the sample pad of the test strip is allowed to migrate to the absorbent body. Thus, the absorbent article can detect an antigen contained in vaginal excretions in a state in which the detection is less susceptible to the influence of urine.

[Aspect B2]

The absorbent article according to Aspect B1, wherein the antigen contained in the vaginal excretions is also contained in urine.

In the absorbent article, the detection target, specifically, the antigen contained in vaginal excretions, is also contained in urine, and thus, urine tends to easily influence the detection of the antigen contained in the vaginal excretions.

In the absorbent article, since the sample pad or conjugate pad is in direct contact with the absorbent body or indirectly in contact with the absorbent body via a liquid-permeable sheet, urine which reaches the sample pad of the test strip is allowed to migrate to the absorbent body. Thus, the absorbent article can detect an antigen contained in vaginal excretions in a state in which the detection is less susceptible to the influence of urine.

[Aspect B3]

The absorbent article according to Aspect B1 or B2, wherein the test strip further comprises, between the deployment layer and the absorbent body, a migration-inhibiting lower layer for inhibiting migration of the excretions of a wearer absorbed by the absorbent body to the deployment layer.

The absorbent article comprises a predetermined migration-inhibiting lower layer, and thus, can inhibit migration of excretions of the wearer absorbed by the absorbent body, such as vaginal excretions, urine, feces, etc., to the deployment layer. As a result, the absorbent article can detect an antigen contained in vaginal excretions in a state in which the detection is less susceptible to the influence of urine.

[Aspect B4]

The absorbent article according to any one of Aspects B1 to B3, wherein the test strip further comprises, between the deployment layer and the top sheet, a migration-inhibiting upper layer for inhibiting migration of excretions of the wearer to the deployment layer.

The absorbent article comprises a predetermined migration-inhibiting upper layer, and thus, can inhibit migration of excretions of the wearer, such as vaginal excretions, urine, feces, etc., directly to the deployment layer without passing through the sample pad and conjugate pad. As a result, the absorbent article can detect an antigen contained in vaginal excretions in a state in which the detection is less susceptible to the influence of excretions.

[Aspect B5]

The absorbent article according to any one of Aspects B1 to B4, wherein the test strip further comprises, between the deployment layer and the top sheet, a migration-inhibiting upper layer for inhibiting migration of excretions of a wearer to the deployment layer, and the test strip further comprises, between the deployment layer and the absorbent body, a migration-inhibiting lower layer for inhibiting migration of excretions absorbed by the absorbent body to the deployment layer, and

the test strip further comprises a seal part for sealing outer edges of the migration-inhibiting upper layer and the migration-inhibiting lower layer.

In the absorbent article, the test strip comprises a migration-inhibiting upper layer, a migration-inhibiting lower layer, and a seal part for sealing the outer edges of these layers, and thus, migration of excretions of the wearer, such as vaginal excretions, urine, feces, etc., directly to the deployment layer without passing through the sample pad and conjugate pad can be inhibited. As a result, the absorbent article can detect an antigen contained in vaginal excretions in a state in which the detection is less susceptible to the influence of excretions.

[Aspect B6]

The absorbent article according to any one of Aspects B1 to B5, wherein a fiber density of the sample pad is greater than a fiber density of the top sheet.

The absorbent article easily allows vaginal excretions to migrate from the top sheet to the sample pad.

[Aspect B7]

The absorbent article according to any one of Aspect B1 to 6, wherein a hydrophilicity of the sample pad is greater than a hydrophilicity of the top sheet.

The absorbent article easily allows vaginal excretions to migrate from the top sheet to the sample pad.

[Aspect B8]

The absorbent article according to any one of Aspects B1 to B7, wherein the sample pad is in contact with the absorbent body, and a fiber density of the absorbent body is greater than a fiber density of the sample pad.

The absorbent article easily allows urine to transfer from the sample pad to the absorbent body.

[Aspect B9]

The absorbent article according to any one of Aspects B1 to 8, wherein the sample pad is in contact with the absorbent body, and a hydrophilicity of the absorbent body is greater than a hydrophilicity of the sample pad.

The absorbent article easily allows urine to transfer from the sample pad to the absorbent body.

[Aspect B10]

The absorbent article according to any one of Aspect B1 to 9, wherein a fiber density of the conjugate pad is greater than a fiber density of the sample pad.

The absorbent article easily allows urine to transfer from the conjugate pad to the absorbent body.

[Aspect B11]

The absorbent article according to any one of Aspect B1 to B10, wherein a hydrophilicity of the conjugate pad is greater than a hydrophilicity of the sample pad.

The absorbent article easily allows urine to transfer from the conjugate pad to the absorbent body.

[Aspect B12]

The absorbent article according to any one of Aspects B1 to B11, wherein the deployment layer is not in direct contact with the absorbent body.

The absorbent article is configured such that the test strip is less susceptible to the influence of excretions.

[Aspect B13]

An absorbent article, comprising a test strip for detecting an antigen contained in vaginal excretions and having a thickness direction, wherein

the absorbent article comprises a liquid-permeable top sheet, the test strip, an absorbent body, and a liquid-impermeable back sheet in that order in the thickness direction, and
the test strip further comprises, between the test strip and the absorbent body, a migration-inhibiting lower layer for inhibiting migration of excretions of a wearer absorbed by the absorbent body to the test strip.

The absorbent article comprises a predetermined migration-inhibiting lower layer, and thus, can inhibit migration of excretions of the wearer absorbed by the absorbent body, such as vaginal excretions, urine, feces, etc., to the test strip. As a result, the absorbent article can detect an antigen contained in vaginal excretions in a state in which the detection is less susceptible to the influence of urine.

[Aspect B14]

The absorbent article according to Aspect B13, wherein the test strip further comprises, between the test strip and the top sheet, a migration-inhibiting upper layer for inhibiting migration of excretions other than vaginal excretions of a wearer to the test strip.

The absorbent article comprises a predetermined migration-inhibiting upper layer, and thus, can inhibit migration of excretions other than the vaginal excretions of the wearer to the test strip. As a result, the absorbent article can detect an antigen contained in vaginal excretions in a state in which the detection is less susceptible to the influence of excretions.

DESCRIPTION OF REFERENCE SIGNS

1 absorbent article (panty liner)
3, 20 top sheet
5 back sheet
7, 20 absorbent body
9 second sheet
21, 60 test strip
23, 64 sample pad (first member)
25 conjugate pad
66A moving part (display moving part)
66B moving part (terminal moving part)
27 deployment membrane
31 migration-inhibiting upper layer
33 migration-inhibiting lower layer
35 cover tape
101 fixing part
L front-rear direction
W width direction
CL center CT of absorbent article in front-rear direction L
CT center CT of test strip in front-rear direction L
SR
S1 front section of absorbent article
S2 rear section of absorbent article
S3 central section of absorbent article

**Claims**

1. An absorbent article, comprising a test strip for detecting luteinizing hormone from vaginal discharge using immunochromatography, wherein
the test strip detects 0.05 mIU/mL to 2.5 mIU/mL of luteinizing hormone as positive.

2. The absorbent article according to claim 1, wherein the test strip has a width of 5 mm or less.

3. The absorbent article according to claim 1, wherein the test strip has a width of 3 mm or less.

4. The absorbent article according to claim 1 or 2, wherein the test strip comprises:

a first member which contacts vaginal discharge,

a second member which contains a labeled antibody that recognizes luteinizing hormone in vaginal discharge, and

a third member which has a display part which contains a capture antibody,

the capture antibody is capable of capturing a complex formed by binding of the labeled antibody and luteinizing hormone, and

the test strip is configured such that the luteinizing hormone in vaginal discharge moves from the first member, through the second member, to the display part of the third member, and that

the display part of the third member changes color when the capture antibody captures the complex.

5. The absorbent article according to claim 4, wherein the first member, the second member, and the third member are stacked in this order at least partially overlapping each other, and

a sum of thicknesses of the first member, the second member, and the third member in the stacking direction is 1.5 mm or less.

6. The absorbent article according to claim 4, wherein the first member is composed of fibers having an average fiber diameter of 0.5 to 5 $\mu$m,

the second member is composed of fibers having an average fiber diameter of 10 to 40 $\mu$m, and

an average fiber diameter of the fibers of the first member is less than an average fiber diameter of the fibers of the second member.

7. The absorbent article according to claim 4, wherein an average fiber diameter R1 of the fibers constituting the first member and an average fiber diameter R2 of the fibers constituting the second member satisfy the relationship $5 \leq (R2/R1) \leq 20$.

8. The absorbent article according to claim 4, which satisfies at least one of the following:

(a) the second member has a basis weight of 100 gsm or more,

(b) a sum of thicknesses of the first member, the second member, and the third member is 1.5 mm or less, and the thickness of the second member accounts for 30% or more of this sum of the thicknesses, and

(b) the second member has a density of 0.05 to 0.80 g/m$^3$.

9. The absorbent article according to claim 4, wherein the labeled antibody comprises a colorant having a particle diameter of 10 nm to 500 nm.

10. The absorbent article according to claim 4, wherein the labeled antibody comprises a colorant having a particle diameter of 250 nm to 500 nm.

11. The absorbent article according to claim 1 or 2, wherein the absorbent article comprises a top sheet, and when the absorbent article is used, the vaginal discharge reaches the test strip through the top sheet.

12. The absorbent article according to claim 11, wherein the detection is performed by directly contacting at least a portion of the top sheet with the vulva.

13. The absorbent article according to claim 11, wherein a basis weight of the top sheet is 20 gsm or less.

14. The absorbent article according to claim 11, wherein a thickness of the top sheet is 1 mm or less.

15. The absorbent article according to claim 11, wherein a single fiber diameter of fibers constituting the top sheet is 8.0 dtex or less.

16. The absorbent article according to claim 11, wherein a basis weight of the top sheet is 5 gsm or more, a thickness of the top sheet is 0.1 mm or more, and a single fiber diameter of fibers constituting the top sheet is 1.0 dtex or more.

17. The absorbent article according to claim 11, wherein the top sheet at least partially has an embossed pattern, and a width of the test strip is greater than a size and/or spacing of each pattern of the embossed pattern.

18. The absorbent article according to claim 11, wherein the top sheet partially has an embossed pattern, and the test strip is capable of contacting the vaginal discharge through an area of the top sheet which does not have the embossed pattern.

19. The absorbent article according to claim 11, wherein the top sheet does not contain $TiO_2$.

20. The absorbent article according to claim 11, wherein the test strip comprises a first member which contacts the vaginal discharge, the first member is arranged in a position which faces a vaginal part of a wearer when worn, and
an average fiber diameter of fibers constituting the sample pad is equal to or less than an average fiber diameter of fibers constituting the top sheet.

21. A test strip for an absorbent article for detecting luteinizing hormone in vaginal discharge using immunochromatography, wherein
0.05 mIU/mL to 2.5 mIU/mL of luteinizing hormone is detected as positive.

# Fig. 1

EXAMPLE 1

(mIU/mL)

0    0.05    0.1    0.2    0.5    1.0

EXAMPLE 2

(mIU/mL)

0    0.05    0.1    0.2    0.5    1.0

# Fig. 2

SENSITIVITY

| [mIU] | 0 | 0.05 | 0.1 | 0.2 | 0.5 | 1 |
|---|---|---|---|---|---|---|
| EXAMPLE 1 | 11.1 | 15.8 | 33.3 | 24.5 | 41.2 | 103.2 |
| EXAMPLE 2 | 13.8 | 26.9 | 41.1 | 60.7 | 85.0 | 177.0 |

COLOR INTENSITY COMPARISON

# Fig. 3-1

Fig. 3-2

SAMPLE PAD

TEST LINE
(DISPLAY PART)

CONTROL LINE
(SECOND DISPLAY PART)

ADSORPTION
PAD

CONJUGATE PAD

MEMBRANE

Fig. 3-3

COMPRESSED PART

TOP SHEET

ADHESIVE

SECOND
SKIN-SIDE SHEET
(WIDE FILM)

TEST MEMBER

SECOND
NON-SKIN-SIDE SHEET
(WIDE FILM)

ADHESIVE

SECOND SHEET
(AIR-THROUGH
NONWOVEN FABRIC)

ABSORBENT CORE
(AIR-LAID PULP)

ADHESIVE

ADHESIVE

T1

T

T2

BACK SHEET

FASTENING
PART

EP 4 607 194 A1

# Fig. 3-4

COLORED REGION

TRANSPARENT FILM
(FIRST SKIN-SIDE SHEET)

COLORED REGION

WIDE FILM (SECOND
SKIN-SIDE SHEET)

ADHESIVE

MEMBRANE
(DISPLAY PART)

WIDE FILM (SECOND
NON-SKIN-SIDE SHEET)

L

TRANSPARENT FILM (FIRST
NON-SKIN-SIDE SHEET)

ADHESIVE

CONJUGATE PAD
(MOVING PART/REACTION PART)

SAMPLE PAD
(CONTACT PART)

T1  T2

T

43

# Fig. 4

URINARY LH (mIU/mg-CRE)　　　　　LEUKORRHEAL LH (mIU/mg)

ID4026　　　　　　78.45　13.28

VAGINAL
DISCHARGE

URINE

NUMBER OF DAYS (DAYS)
BASED ON URINARY LH SURGE

LEUKORRHEAL LH AND URINARY LH SURGED
ON THE SAME DAY

# Fig. 5

URINARY LH (mIU/mg-CRE)　　　　　LEUKORRHEAL LH (mIU/mg)

ID4039

24.49　0.69

VAGINAL
DISCHARGE

URINE

NUMBER OF DAYS (DAYS)
BASED ON URINARY LH SURGE

LEUKORRHEAL LH SURGED 1 DAY LATER
THAN URINARY LH

# Fig. 6

LEUKORRHEAL
LH SURGE
(mIU/mg)

LH CONCENTRATION AROUND DAY
OF LEUKORRHEAL LH SURGE

0.15mIU/mg

NUMBER OF DAYS FROM LEUKORRHEAL LH SURGE

# Fig. 7

ROC CURVE

AUC=0.79

TPF

FPF

## Fig. 8-1

**4020**

Fig. 8-2

Fig. 8-3

Fig. 9

EP 4 607 194 A1

Fig. 10

EP 4 607 194 A1

Fig. 11

EP 4 607 194 A1

# Fig.12

Fig. 13

## INTERNATIONAL SEARCH REPORT

| International application No. |
| --- |
| **PCT/JP2023/037940** |

**A. CLASSIFICATION OF SUBJECT MATTER**

*G01N 33/53*(2006.01)i; *A61F 13/42*(2006.01)i; *A61F 13/511*(2006.01)i; *G01N 33/543*(2006.01)i
FI: G01N33/53 B; G01N33/543 521; A61F13/511 300; A61F13/511 100; A61F13/42 B

According to International Patent Classification (IPC) or to both national classification and IPC

**B. FIELDS SEARCHED**

Minimum documentation searched (classification system followed by classification symbols)

G01N33/53; A61F13/42; A61F13/511; G01N33/543

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2021/132724 A1 (UNICHARM CORPORATION) 01 July 2021 (2021-07-01)<br>paragraphs [0048]-[0060], [0081]-[0082], fig. 1, 3, 6 | 1-21 |
| A | WO 2016/002743 A1 (NIPPON STEEL & SUMIKIN CHEMICAL CO., LTD.) 07 January 2016 (2016-01-07)<br>paragraphs [0005]-[0026] | 1-21 |
| A | JP 8-285849 A (MOCHIDA PHARMACEUT CO LTD) 01 November 1996 (1996-11-01)<br>paragraphs [0022]-[0025], [0064]-[0067], [0089]-[0094] | 1-21 |
| A | WO 2020/053966 A1 (UNICHARM CORPORATION) 19 March 2020 (2020-03-19)<br>paragraphs [0033]-[0050] | 1-21 |
| A | JP 2003-517584 A (THE PROCTER & GAMBLE COMPANY) 27 May 2003 (2003-05-27)<br>entire text, all drawings | 1-21 |

☐ Further documents are listed in the continuation of Box C.  ☑ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **06 December 2023** | **19 December 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

# EP 4 607 194 A1

## INTERNATIONAL SEARCH REPORT
### Information on patent family members

International application No.

**PCT/JP2023/037940**

| Patent document cited in search report | | | Publication date (day/month/year) | Patent family member(s) | | | Publication date (day/month/year) |
|---|---|---|---|---|---|---|---|
| WO | 2021/132724 | A1 | 01 July 2021 | (Family: none) | | | |
| WO | 2016/002743 | A1 | 07 January 2016 | US 2017/0168049 A1 entire text, all drawings EP 3165923 A1 CN 106662585 A | | | |
| JP | 8-285849 | A | 01 November 1996 | (Family: none) | | | |
| WO | 2020/053966 | A1 | 19 March 2020 | JP 2020-39570 A entire text, all drawings JP 6445732 B1 CN 112672721 A KR 10-2021-0041050 A | | | |
| JP | 2003-517584 | A | 27 May 2003 | US 6093869 A entire text, all drawings WO 2000/065096 A1 | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007526443 A
- WO 2021132724 A
- WO 2021132724 A1
- JP 5087807 A

**Non-patent literature cited in the description**

- *Bioeng. Transl. Med.*, 2017, vol. 2 (3), 238-246
- Monitoring the Menstrual Cycle: Comparison of Urinary and Serum Reproductive Hormones Referenced True Ovulation. *The European Journal of Contraception and Reproductive Health Care*, 2015, 1-13
- Characteristics of Urinary Luteinizing Hormone Surge in Young Ovulatory Women. *Fertil. Steril.*, September 2007, vol. 88 (3), 684-90
- **D. B. DUNSON et al.** Day-Specific Probabilities of Clinical Pregnancy Based on Two Studies with Perfect Measurements of Ovulation. *Human Reproduction*, 1999, vol. 14 (7), 1835-1839